(19) Europäisches Patentamt
European Patent Office
Office européen des brevets

(11) EP 1 813 615 A1

(12) # EUROPEAN PATENT APPLICATION
published in accordance with Art. 158(3) EPC

(43) Date of publication:
**01.08.2007 Bulletin 2007/31**

(21) Application number: **05795682.3**

(22) Date of filing: **24.10.2005**

(51) Int Cl.:
***C07D 487/22*** *(2006.01)* ***G02B 5/00*** *(2006.01)*
***G02B 5/22*** *(2006.01)* ***G02B 5/30*** *(2006.01)*

(86) International application number:
**PCT/JP2005/019475**

(87) International publication number:
**WO 2006/046497 (04.05.2006 Gazette 2006/18)**

(84) Designated Contracting States:
**DE FR GB NL**

(30) Priority: **25.10.2004 JP 2004310118**
**25.10.2004 JP 2004310117**
**13.12.2004 JP 2004359249**

(71) Applicant: **Mitsui Chemicals, Inc.**
**Tokyo 105-7117 (JP)**

(72) Inventors:
- **MINAMI, Hiromi,**
**Mitsui Chemicals, Inc.**
**Sodegaura-shi, Chiba 2990265 (JP)**
- **OKAMURA, Tomoyuki,**
**Mitsui Chemicals, Inc.**
**Sodegaura-shi, Chiba 2990265 (JP)**
- **KAGAYAMA, Akifumi,**
**Mitsui Chemicals, Inc.**
**Sodegaura-shi, Chiba 2990265 (JP)**
- **SUZIKI, Rihoko,**
**Mitsui Chemicals, Inc.**
**Sodegaura-shi, Chiba 2990265 (JP)**
- **FUJII, Kenichi,**
**Mitsui Chemicals, Inc.**
**Sodegaura-shi, Chiba 2990265 (JP)**
- **OI, Ryu,**
**Du Pont-Mitsui Polychemicals Co., Ltd.**
**Minato-ku, Tokyo 1057117 (JP)**
- **NISHIMOTO, Taizo,**
**Mitsui Chemicals, Inc.**
**Sodegaura-shi, Chiba 2990265 (JP)**
- **MOROHASHI, Makoto,**
**Mitsui Chemicals, Inc.**
**Sodegaura-shi, Chiba 2990265 (JP)**
- **MISAWA, Tsutami,**
**Mitsui Chemicals, Inc.**
**Minato-ku, Tokyo 1057117 (JP)**
- **FUKUDA, Shin,**
**Mitsui Chemicals, Inc.**
**Sodegaura-shi, Chiba 2990265 (JP)**

(74) Representative: **HOFFMANN EITLE**
**Patent- und Rechtsanwälte**
**Arabellastrasse 4**
**81925 München (DE)**

(54) # OPTICAL FILTER AND ITS APPLICATIONS, AND PORPHYRIN COMPOUND USED IN OPTICAL FILTER

(57) An optical filter of the present invention contains at least one tetra-acenaphthoporphyrin compound. The tetra-acenaphthoporphyrin compound has preferably its absorption maximum in a wavelength range of from 530 to 570 nm and a ratio of an absorption coefficient at 550 nm to an absorption coefficient at 525 nm of not less than 1.2. Also, a percentage of light transmittance at 550 nm to light transmittance at 525 nm is preferably not more than 85%.

According to the present invention, it is possible to provide the optical filter which can eliminate an unnecessary light mainly existing in a wavelength of from 520 to 700 nm and is excellent in durability. A display unit using the optical filter of the present invention can improve contrast in a bright place, and improve color purity of luminescent color.

EP 1 813 615 A1

## Description

### TECHNICAL FIELD

The present invention relates to an optical filter and a porphyrin compound used in the optical filter. More particularly, the present invention relates to an optical filter capable of realizing a display unit which is excellent in durability, and is particularly excellent in contrast in a bright place and color purity of luminescent color, and a porphyrin compound used in the optical filter.

### BACKGROUND ART

In late years, with the sophisticated society, optoelectronics-related components and equipments have been remarkably advanced day by day. Among others, a display presenting pictures has been remarkably spread and developed for use in computer monitor devices and the like, in addition to conventional television receivers. With the development of these devices, particularly, a large-scale, thinned, and high quality display has been in high demand from the market.
In recent years, when they are used as a display, full color display is natural. In screen display units such as a liquid crystal display (LCD), a plasma display panel (PDP), an electroluminescence display (ELD), a cathode ray tube (CRT), fluorescent display tube, a field emission display and the like, color images are displayed in combination of so-called three primary colors such as red, green and blue colors.
However, in a bright place such as a fluorescent lamp or the like, the contrast of a display unit is generally decreased due to external light. This phenomenon is attributable to the fact that light of a fluorescent lamp is reflected on a front surface of the display unit and/or inside the display unit so that the luminance of black image is enhanced. As a result, an image with lost contrast inherent in the display unit is visualized.
In response to this, there has been conventionally attempted to reduce the influence of external light and enhance the contrast by using a ND filter (refer to Patent Document 1).
However, in this method, since a necessary emission emitted from the display unit is also cut off at the same time, luminance might also be decreased. If external light is selectively cut off and only necessary light from the display unit is allowed to pass through, an ideal optical filter is obtained.
Furthermore, emission of extra light for reducing the color purity, which is called an unnecessary emission, is included in any of image display units, in addition to emission in a wavelength of red, green and blue colors from its principle of emission. Color reproductivity (color purity) of an image is lost by this unnecessary emission. If light in an unnecessary wavelength is cut off and a single color can be taken out from three primary colors of red, green and blue colors respectively, the color purity of the image can be enhanced.
In response to this, various methods have been studied in order to improve the color purity, but its effects are not sufficient (refer to Patent Documents 2 to 6).
Methods for improving the contrast and the color purity of the aforementioned display unit at the same time include a method using a filter containing a dye absorbing light in an unnecessary wavelength region. Various reviews have hitherto been made. For example, a dye eliminating an unnecessary light between green and red colors has been reported (refer to Patent Documents 7 and 8).
However, even though a filter for effectively eliminating an unnecessary light mainly existing in the vicinity of 550 nm contained in a fluorescent lamp or the like due to a dye has been highly in demand from the market, the reality is that such a filter has not been put into practical use. This is attributable to the fact that performance of the dye capable of absorbing an unnecessary light as described above is remarkably deteriorated under the conditions including environment of use and/or temperature, light, heat and the like caused by the display unit. In particular, there has been a problem in durability when a dye is used in an adhesive.
For that reason, there has been expected appearance of a technique for providing sufficient durability and eliminating an unnecessary light existing in the vicinity of 550 nm.
Patent Document 1: Japanese Patent Laid-open No. 11-259006
Patent Document 2: Japanese Patent Laid-open No. 58-153904
Patent Document 3: Japanese Patent Laid-open No. 59-221943
Patent Document 4: Japanese Patent Laid-open No. 60-22102
Patent Document 5: Japanese Patent Laid-open No. 60-65050
Patent Document 6: Japanese Patent Laid-open No. 5-316329
Patent Document 7: Japanese Patent Laid-open No. 2002-363434
Patent Document 8: Japanese Patent Laid-open No. 2003-167118

DISCLOSURE OF THE INVENTION

It is an object of the present invention to provide an optical filter which is capable of eliminating an unnecessary emission of a display unit and an unnecessary light mainly existing in a wavelength range of from 530 to 570 nm contained in external light without preventing a necessary emission of the display unit, and which is excellent in durability as well. In particular, it is an object of the present invention to provide an optical filter capable of realizing a display unit which is excellent in contrast in a bright place and color purity of luminescent color.

The present inventors have been conducted an extensive study, and as a result, have found that the above object can be solved by an optical filter containing a tetra-acenaphthoporphyrin compound. Thus, the present invention has been completed.
That is, an optical filter according to the present invention contains at least one tetra-acenaphthoporphyrin compound.
The aforementioned tetra-acenaphthoporphyrin compound has preferably an absorption maximum in a wavelength range of from 530 to 570 nm and a ratio of an absorption coefficient at a wavelength of 550 nm to an absorption coefficient at a wavelength of 525 nm of not less than 1.2.

A percentage of light transmittance at a wavelength of 550 nm to light transmittance at a wavelength of 525 nm is preferably not more than 85%.
Furthermore, the optical filter preferably contains a dye (A) having an absorption maximum in a wavelength range of from 440 to 510 nm and/or a dye (B) having an absorption maximum in a wavelength range of from 575 to 620 nm.

It is preferable that the optical filter has an electrically conductive layer (D) and/or a functional transparent layer (E), or it is preferable that the optical filter has a toning layer (F).
The above tetra-acenaphthoporphyrin compound is preferably a compound represented by the following general formula (3-1),

[Chemical Formula 1]

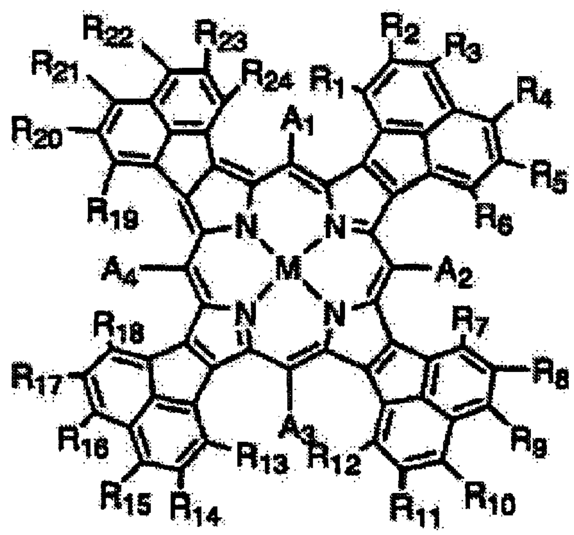

(3-1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $A_1$ to $A_4$ each independently represent a hydrogen atom, an alkyl group which may be substituted, an aryl group which may be substituted, a heteroaryl group which may be substituted, an alkenyl group which may be substituted or an alkynyl group which may be substituted; and M represents two hydrogen atoms, a divalent metal atom, or a trivalent or tetravalent metallic derivative.

The plasma display according to the present invention includes the optical filter as described above.

The liquid crystal display according to the present invention includes the optical filter as described above. The organic electroluminescence display according to the present invention includes the optical filter as described above. Furthermore, the optical filter according to the present invention contains a dye which has an absorption maximum in a wavelength range of from 530 to 570 nm and a ratio of an absorption coefficient at a wavelength of 550 nm to an absorption coefficient at a wavelength of 525 nm of not less than 1.2, and which has an absorption retention rate represented by the following equation (1) of not less than 80% when a visible light ray of from 380 to 780 nm is irradiated at 50 mW/cm$^2$ for 250 hours on an acrylic adhesive containing the dye,

$$\text{Absorption retention rate (\%)} = (T_0 - T_{max,1})/(T_0 - T_{max,0}) \times 100 \quad (1)$$

wherein To represents an average light transmittance (%) of light transmittances at from 380 to 780 nm that are constant in a wavelength range of 30 nm; $T_{max,0}$ represents the light transmittance (%) at a maximum absorption wavelength before a visible light ray is irradiated; and $T_{max,1}$ represents the light transmittance (%) at a maximum absorption wavelength after a visible light ray is irradiated for 250 hours.

The tetra-acenaphthoporphyrin compound according to the present invention is represented by the following general formula (1-1),

[Chemical Formula 2]

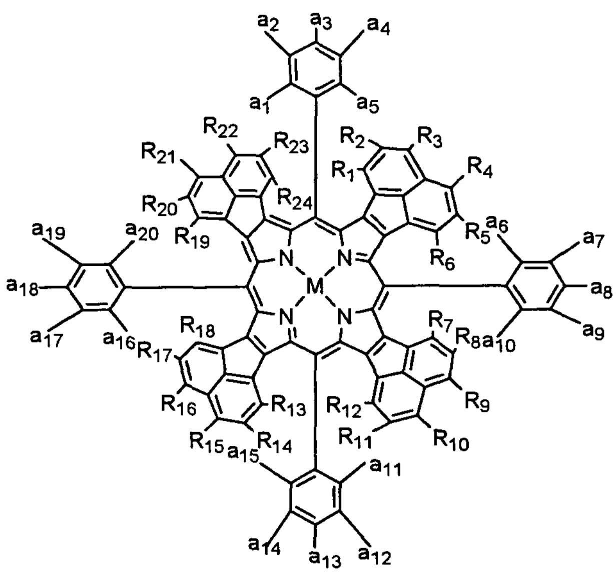

(1 − 1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $a_1$ to $a_{20}$ each independently represent a hydrogen atom, a halogen atom, an alkyl group which may be substituted, an aryl group which may be substituted or an alkoxy group which may be substituted, with the proviso that, in each of $a_1$ to $a_5$, $a_6$ to $a_{10}$, $a_{11}$ to $a_{15}$ and $a_{16}$ to $a_{20}$, one or more are an alkoxy group having 4 to 20 carbon atoms which may be substituted;

and M represents two hydrogen atoms, a divalent metal atom, or a trivalent or tetravalent metallic derivative.
Further, the tetra-acenaphthoporphyrin compound according to the present invention is represented by the following general formula (2-1),

[Chemical Formula 3]

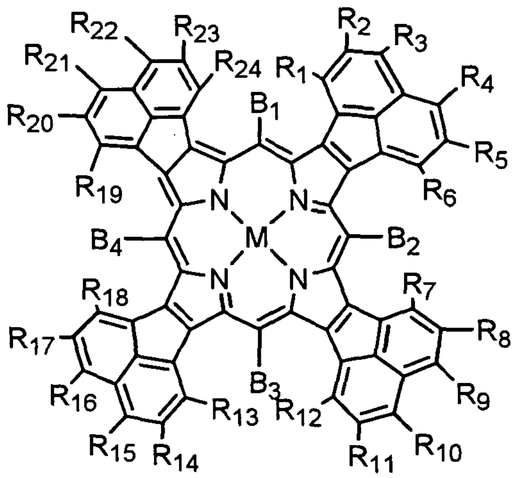

(2 − 1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $B_1$ to $B_4$ each independently represent an alkenyl group which may be substituted, an alkynyl group which may be substituted or a heteroaryl group which may be substituted; and M represents two hydrogen atoms, a divalent metal atom, or a trivalent or tetravalent metallic derivative.
Furthermore, the tetra-acenaphthoporphyrin compound according to the present invention is represented by the following general formula (2-2),

[Chemical Formula 4]

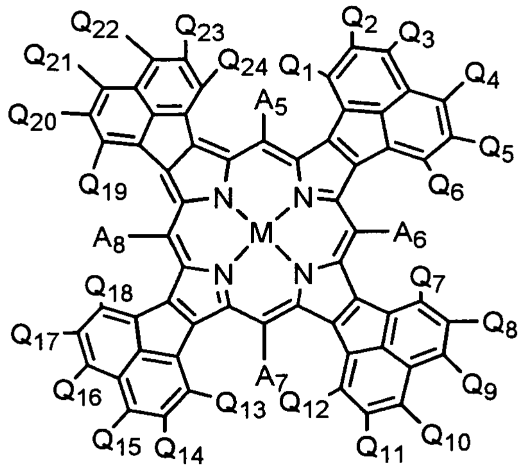

(2－2)

wherein $Q_1$ to $Q_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted, with the proviso that one or more of $Q_1$ to $Q_{24}$ are a nitro group, a cyano group, a hydroxyl group, a carboxyl group, a sulfonic acid group, an alkoxycarbonyl group which may be substituted, an aryloxycarbonyl group which may be substituted, an aminocarbonyl group which may be substituted, an amino group which may be substituted, an aminosulfonyl group which may be substituted, an alkynyl group which may be substituted or a heteroaryl group which may be substituted; $A_5$ to $A_8$ each independently represent a hydrogen atom, an alkyl group which may be substituted or an aryl group which may be substituted; and M represents two hydrogen atoms, a divalent metal atom, or a trivalent or tetravalent metallic derivative.

## EFFECT OF THE INVENTION

The optical filter of the present invention is capable of reducing an unnecessary light mainly existing in a wavelength of from 520 to 700 nm and is also excellent in durability. For this reason, the optical filter is considered to be particularly suitable as an optical filter for consumer use in which durability is valued. Further, in a display unit using the optical filter of the present invention, contrast in a bright place can be improved and at the same time color purity of luminescent color can be improved. Accordingly, industrial value of the present invention is great.
Furthermore, the tetra-acenaphthoporphyrin compound of the present invention is excellent in light resistance, heat resistance and wet heat resistance, and has a selective absorption, that is, an absorption maximum in a wavelength of from 520 to 700 nm in a base material. So the compound is suitably used for an optical filter.

## BEST MODE FOR CARRYING OUT THE INVENTION

The present invention will be described in more detail below.
The optical filter according to the present invention contains at least one tetra-acenaphthoporphyrin compound in a component member. It is preferable to use a tetra-acenaphthoporphyrin compound having an absorption maximum in a wavelength range of from 520 to 700 nm. The above compound having an absorption maximum preferably in a wavelength range of from 520 to 620 nm, more preferably in a wavelength range of from 530 to 570 nm is favorably used.
Furthermore, the aforementioned tetra-acenaphthoporphyrin has an absorption maximum in the above wavelength range, and a ratio of an absorption coefficient at a wavelength of 550 nm to an absorption coefficient (JIS K0115) at a wavelength of 525 nm in a solvent is preferably not less than 1.2, more preferably not less than 1.5, particular

preferably not less than 1.8. When the plasma display panel or the like has a peak of green color emission in the vicinity of 525 nm and the ratio of the absorption coefficients is not less than 1.2, only an unnecessary light existing in the vicinity of 550 nm to be described later can be reduced with good efficiency without deteriorating the luminance of a display unit.

As the above tetra-acenaphthoporphyrin compound, a compound represented by the above general formula (3-1) can be preferably used.

The substituent of the tetra-acenaphthoporphyrin compound represented by the above formula (3-1) is not particularly limited, but concrete examples are illustrated below.

In the compound represented by the above formula (3-1), $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted.

Examples of the halogen atom include fluorine, chlorine, bromine and iodine.

Examples of the alkyl group which may be substituted include a linear, branched or cyclic alkyl group having not more than 20 carbon atoms such as a methyl group, an ethyl group, an n-propyl group, an iso-propyl group, an n-butyl group, an iso-butyl group, a sec-butyl group, a t-butyl group, an n-pentyl group, an iso-pentyl group, a 2-methylbutyl group, a 1-methylbutyl group, a neo-pentyl group, a 1,2-dimethylpropyl group, a 1,1-dimethylpropyl group, a cyclo-pentyl group, an n-hexyl group, a 4-methylpentyl group, a 3-methylpentyl group, a 2-methylpentyl group, a 1-methylpentyl group, a 3,3-dimethylbutyl group, a 2,3-dimethylbutyl group, a 1,3-dimethylbutyl group, a 2,2-dimethylbutyl group, a 1,2-dimethylbutyl group, a 1,1-dimethylbutyl group, a 3-ethylbutyl group, a 2-ethylbutyl group, a 1-ethylbutyl group, a 1,2,2-trimethylbutyl group, a 1,1,2-trimethylbutyl group, a 1-ethyl-2-methylpropyl group, a cyclo-hexyl group, an n-heptyl group, a 2-methylhexyl group, a 3-methylhexyl group, a 4-methylhexyl group, a 5-methylhexyl group, a 2,4-dimethylpentyl group, an n-octyl group, a 2-ethylhexyl group, a 2,5-dimethylhexyl group, a 2,5,5-trimethylpentyl group, a 2,4-dimethylhexyl group, a 2,2,4-trimethylpentyl group, a 3,5,5-trimethylhexyl group, an n-nonyl group, an n-decyl group, a 4-ethyloctyl group, a 4-ethyl-4,5-dimethylhexyl group, an n-undecyl group, an n-dodecyl group, a 1,3,5,7-tetraethyloctyl group, a 4-butyloctyl group, a 6,6-diethyloctyl group, an n-tridecyl group, a 6-methyl-4-butyloctyl group, an n-tetradecyl group, an n-pentadecyl group, a 3,5-dimethylheptyl group, a 2,6-dimethylheptyl group, a 2,4-dimethylheptyl group, a 2,2,5,5-tetramethylhexyl group, a 1-cyclo-pentyl-2,2-dimethylpropyl group, a 1-cyclo-hexyl-2,2-dimethylpropyl group and the like;

an aralkyl group such as a benzyl group, a nitrobenzyl group, a cyanobenzyl group, a hydroxybenzyl group, a methylbenzyl group, a dimethylbenzyl group, a trimethylbenzyl group, a dichlorobenzyl group, a methoxybenzyl group, an ethoxybenzyl group, a trifluoromethylbenzyl group, a naphthylmethyl group, a nitronaphthylmethyl group, a cyanonaphthylmethyl group, a hydroxynaphthylmethyl group, a methylnaphthylmethyl group, a trifluoromethylnaphthylmethyl group and the like;

a halogenoalkyl group such as a trifluoromethyl group, a heptafluoropropyl group and the like;

a hydroxyalkyl group such as a hydroxyethyl group, a hydroxypropyl group and the like;

an alkoxyalkyl group such as a methoxymethyl group, a methoxyethyl group, an ethoxyethyl group, an iso-propyloxyethyl group, a 3-methoxypropyl group, a 2-methoxybutyl group and the like; and

an aryloxyalkyl group such as a phenoxymethyl group, a phenoxyethyl group and the like.

Examples of the aryl group which may be substituted include an aryl group such as a phenyl group, a nitrophenyl group, a cyanophenyl group, a hydroxyphenyl group, a methylphenyl group, a dimethylphenyl group, a trimethylphenyl group, a dichlorophenyl group, a methoxyphenyl group, an ethoxyphenyl group, a trifluoromethylphenyl group, an N,N-dimethylaminophenyl group, a naphthyl group, a methylnaphthyl group, a nitronaphthyl group, a cyanonaphthyl group, a hydroxynaphthyl group, a trifluoromethylnaphthyl group and the like.

Examples of the heteroaryl group which may be substituted include a pyrrolyl group, a thienyl group, a furanyl group, an oxazoyl group, an isoxazoyl group, an oxadiazoyl group, an imidazoyl group, a benzooxazoyl group, a benzothiazoyl group, a benzoimidazoyl group, a benzofuranyl group, an indoyl group and the like.

Examples of the alkoxy group which may be substituted include an alkoxy group such as a methoxy group, an ethoxy group, an n-propoxy group, an iso-propoxy group, an n-butoxy group, an iso-butoxy group, a sec-butoxy group, a t-butoxy group, an n-pentoxy group, an iso-pentoxy group, a neo-pentoxy group, an n-hexyloxy group, an n-dodecyloxy group and the like; an alkoxyalkoxy group such as a methoxyethoxy group, an ethoxyethoxy group, a 3-methoxypropyloxy group, a 3-(iso-propyloxy)propyloxy group and the like; and a hydroxyalkoxy group such as a hydroxyethoxy group and the like.

Examples of the aryloxy group which may be substituted include a phenoxy group, a 2-methylphenoxy group, a 4-methylphenoxy group, a 4-t-butylphenoxy group, a 4-iso-propylphenoxy group, a 2-methoxyphenoxy group, a 4-ethoxyphenoxy group, a 4-chlorophenoxy group, a 4-hydroxyphenoxy group and the like.
Examples of the alkylcarbonyl group which may be substituted include an acetyl group, an ethylcarbonyl group, an n-propylcarbonyl group, an iso-propylcarbonyl group, an n-butylcarbonyl group, an iso-butylcarbonyl group, a sec-butylcarbonyl group, a t-butylcarbonyl group, an n-pentylcarbonyl group, an iso-pentylcarbonyl group, a neo-pentylcarbonyl group, a 2-methylbutylcarbonyl group, a nitrobenzylcarbonyl group and the like.

Examples of the arylcarbonyl group which may be substituted include a phenylcarbonyl group, a 4-t-butylphenylcarbonyl group and the like.
Examples of the alkoxycarbonyl group which may be substituted include a methoxycarbonyl group, an ethoxycarbonyl group, an isopropyloxycarbonyl group, a 2,4-dimethylbutyloxycarbonyl group and the like.

Examples of the aryloxycarbonyl group which may be substituted include a phenoxycarbonyl group, a 4-t-butylphenoxycarbonyl group and the like.
Examples of the aminocarbonyl group which may be substituted include an alkylaminocarbonyl group such as an aminocarbonyl group, a methylaminocarbonyl group, an ethylaminocarbonyl group, an n-propylaminocarbonyl group, an n-hexylaminocarbonyl group and the like; a dialkylaminocarbonyl group such as a dimethylaminocarbonyl group, a diethylaminocarbonyl group, a di-n-propylaminocarbonyl group, a di-n-butylaminocarbonyl group, an N-methyl-N-cyclohexylaminocarbonyl group and the like; an arylaminocarbonyl group such as a phenylaminocarbonyl group, a 4-methylphenylaminocarbonyl group, a 2-methoxyphenylaminocarbonyl group, a 4-n-propylphenylaminocarbonyl group and the like; and a diarylaminocarbonyl group such as a diphenylaminocarbonyl group, a di (4-methylphenyl) aminocarbonyl group and the like.

Examples of the amino group which may be substituted include an alkylamino group such as an amino group, a methylamino group, an ethylamino group, a propylamino group, a butylamino group and the like; a dialkylamino group such as a dimethylamino group, a diethylamino group, a dipropylamino group, a dibutylamino group and the like; an alkylcarbonylamino group such as an acetylamino group, an ethylcarbonylamino group, a butylcarbonylamino group and the like; an arylcarbonylamino group such as a phenylcarbonylamino group, a 4-ethylphenylcarbonylamino group, a 3-butylphenylcarbonylamino group and the like; an alkoxycarbonylamino group such as a methoxycarbonylamino group and the like; and an aryloxycarbonylamino group such as a phenoxycarbonylamino group and the like.

Examples of the alkylthio group which may be substituted include a methylthio group, an ethylthio group, an n-propylthio group, an iso-propylthio group, an n-butylthio group, an iso-butylthio group, a sec-butylthio group, a t-butylthio group, an n-pentylthio group, an iso-pentylthio group, a 2-methylbutylthio group, a 1-methylbutylthio group, a neo-pentylthio group, a 1,2-dimethylpropylthio group, a 1,1-dimethylpropylthio group and the like.

Examples of the arylthio group which may be substituted include a phenylthio group, a 4-methylphenylthio group, a 2-methoxyphenylthio group, a 4-t-butylphenylthio group and the like.
Examples of the aminosulfonyl group which may be substituted include an alkylaminosulfonyl group such as an aminosulfonyl group, a methylaminosulfonyl group, an ethylaminosulfonyl group, an n-propylaminosulfonyl group, an n-hexylaminosulfonyl group and the like; a dialkylaminosulfonyl group such as a dimethylaminosulfonyl group, a diethylaminosulfonyl group, a di-n-propylaminosulfonyl group, a di-n-butylaminosulfonyl group, an N-methyl-N-cyclohexylaminosulfonyl group and the like; an arylaminosulfonyl group such as a phenylaminosulfonyl group, a 4-methylphenylaminosulfonyl group, a 2-methoxyphenylaminosulfonyl group, a 4-n-propylphenylaminosulfonyl group and the like; and a diarylaminosulfonyl group such as a diphenylaminosulfonyl group, a di (4-methylphenyl) aminosulfonyl group and the like.

The alkenyl groups which may be substituted include a substituted or unsubstituted ethenyl group. Examples of the substituent of the ethenyl group at the 2,2'-positions include an alkyl group which may be substituted, an aryl group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted and a trialkylsilyl group.

Concrete examples of the alkenyl group which may be substituted include an ethenyl group, a vinyl group, a propenyl group, a 1-butenyl group, an iso-butenyl group, a 1-pentenyl group, an iso-butenyl group, a 1-pentenyl group, a 2-pentenyl group, a 2-methyl-1-butenyl group, a 3-methyl-1-butenyl group, a 2-methyl-2-butenyl group, a 2,2-dicyanovinyl group, a 2-cyano-2-methylcarboxylvinyl group, a 2-cyano-2-methylsulfonevinyl group and the like.

The alkynyl groups which may be substituted include a substituted or unsubstituted ethinyl group. Examples of the substituent of the ethinyl group at the 2-positions include an alkyl group which may be substituted, an aryl group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted and a trialkylsilyl group.

Concrete examples of the alkynyl group which may be substituted include an ethinyl group, a phenylethinyl group, a 4-methylphenylethinyl group, a 4-methoxyphenylethinyl group, a trimethylsilylethinyl group and the like.
As $R_1$ to $R_{24}$, preferred are a hydrogen atom, a halogen atom, a nitro group, an alkyl group which may be substituted, an aryl group which may be substituted, an alkoxy group which may be substituted and an aryloxy group which may be substituted from the viewpoints of solubility, absorption wavelength, sharpness of absorption peak and easiness of

production of the above compound.

Of $R_1$ to $R_{24}$, $R_1$, $R_2$, $R_5$ to $R_8$, $R_{11}$ to $R_{14}$, $R_{17}$ to $R_{20}$, $R_{23}$ and $R_{24}$ are preferably a hydrogen atom, while $R_3$, $R_4$, $R_9$, $R_{10}$, $R_{15}$, $R_{16}$, $R_{21}$ and $R_{22}$ are preferably a hydrogen atom, a halogen atom, a nitro group, an alkyl group which may be substituted or an alkoxy group which may be substituted.

In the compound represented by the above general formula (3-1), $A_1$ to $A_4$ each independently represent a hydrogen atom, an alkyl group which may be substituted, an aryl group which may be substituted, a heteroaryl group which may be substituted, an alkenyl group which may be substituted or an alkynyl group which may be substituted. Since the substituents of $A_1$ to $A_4$ have an influence on absorption wavelength of a dye, a substituent is preferably selected depending on the desired absorption wavelength. Concrete examples of $A_1$ to $A_4$ and groups which may be substituted with these include those as described above.

The substituents of $A_1$ to $A_4$ are preferably an alkyl group which may be substituted, an aryl group which may be substituted, a heteroaryl group which may be substituted, an alkenyl group which may be substituted or an alkynyl group which may be substituted, more preferably an aryl group which may be substituted, a heteroaryl group which may be substituted, an alkenyl group which may be substituted or an alkynyl group which may be substituted, and further preferably an aryl group which may be substituted, from the viewpoints of the absorption wavelength, easiness of availability of raw materials and solubility.

As the above aryl group which may be substituted, preferred is a phenyl group having a substituent. As the substituent, preferred are an alkyl group and an alkoxy group, and more preferred is an alkoxy group. As the substituents of $A_1$ to $A_4$, a phenyl group which is substituted with an alkoxy group having 4 to 20 carbon atoms is the most preferable.

In the compound represented by the above general formula (3-1), M represents two hydrogen atoms, a divalent metal atom, or a trivalent or tetravalent metallic derivative.

Examples of the:divalent metal atom represented by M include Cu(II), Zn(II), Fe(II), Co(II), Ni(II), Ru(II), Rh(II), Pd(II), Pt(II), Mn(II), Mg(II), Ti(II), Be(II), Ca(II), Ba(II), Cd(II), Hg(II), Pb(II), Sn(II) and the like.

Examples of the trivalent mono-substituted metallic derivative include Al-F, Al-Cl, Al-Br, Al-I, Ga-F, Ga-Cl, Ga-Br, Ga-I, In-F, InCl, In-Br, In-I, Tl-F, Tl-Cl, Tl-Br, Tl-I, Al-$C_6H_5$, Al-$C_6H_4(CH_3)$, In-$C_6H_5$, In-$C_6H_9(CH_3)$, Mn (OH), $Mn(OC_6H_5)$, $Mn[OSi(CH_3)_3]$, Fe-Cl, Ru-Cl and the like. In the present specification, the atomic group having a metal as described above is also simply referred to as an atom.

Examples of the tetravalent di-substituted metallic derivative include $CrCl_2$, $SiF_2$, $SiCl_2$, $SiBr_2$, $SiI_2$, $SnF_2$, $SnCl_2$, $SnBr_2$, $ZrCl_2$, $GeF_2$, $GeCl_2$, $GeBr_2$, $GeI_2$, $TiF_2$, $TiCl_2$, $TiBr_2$, $Si(OH)_2$, $Sn(OH)_2$, $Ge(OH)_2$, $Zr(OH)_2$, $Mn(OH)_2$, $TiA_2$, $CrA_2$, $SiA_2$, $SnA_2$, $GeA_2$ [A represents an alkyl group, a phenyl group, a naphthyl group or derivatives thereof.], $Si(OA')_2$, $Sn(OA')_2$, $Ge(OA')_2$, $Ti(OA')_2$, $Cr(OA')_2$ [A' represents an alkyl group, a phenyl group, a naphthyl group, a trialkylsilyl group, a dialkylalkoxysilyl group or derivatives thereof.], $Si(SA'')_2$, $Sn(SA'')_2$, $Ge(SA'')_2$ [A" represents an alkyl group, a phenyl group, a naphthyl group or derivatives thereof.] and the like. Examples of the oxy metal include VO, MnO, TiO and the like.

In the present invention, M is preferably Cu (II), Zn (II), Fe(II), Co(II), Ni(II), Ru(II), Rh(II), Pd(II), Pt(II), Sn(II), VO, TiO, $Si(Y)_2$ and $Ge(Y)_2$ (Y represents a halogen atom, an alkoxy group, an aryloxy group, an acyloxy group, a hydroxy group, an alkyl group, an aryl group, an alkylthio group, an arylthio group, a trialkylsilyloxy group, a trialkyltinoxy group or a trialkylgermaniumoxy group), and more preferably Cu(II), Co(II), Ni(II), Pd(II), Pt(II) and VO, from the standpoints of light resistance and heat resistance.

The above tetra-acenaphthoporphyrin compound may be used in combination of two or more kinds.

Furthermore, the tetra-acenaphthoporphyrin compound represented by the above formula (1-1), (2-1) or (2-2) is suitably used for an optical filter. Because the compound represented by the above formula (1-1) has a selective absorption, that is, an absorption maximum in a wavelength of from 520 to 620 nm in a base material, while, similarly, the compound represented by the above formulae (2-1) and (2-2) has an absorption maximum in a wavelength of from 520 to 700 nm in a base material. Further, the compound represented by the above formula (1-1) is excellent in solubility.

In the compound represented by the above formula (1-1), $R_1$ to $R_{24}$ have each the same meanings as those in $R_1$ to $R_{24}$ in the above formula (3-1) and its preferable range is also the same. Further, M has the same meanings as those in M in the above formula (3-1) and its preferable range is also the same.

In the above formula (1-1), $a_1$ to $a_{20}$ each independently represent a hydrogen atom, a halogen atom, an alkyl group which may be substituted, an aryl group which may be substituted or an alkoxy group which may be substituted. However, one or more of $a_1$ to $a_5$, $a_6$ to $a_{10}$, $a_{11}$ to $a_{15}$ and $a_{16}$ to $a_{20}$ respectively is an alkoxy group having 4 to 20 carbon atoms which may be substituted.

Here, concrete examples of the halogen atom, the alkyl group which may be substituted, the aryl group which may be substituted and the alkoxy group which may be substituted are the same as those described above.

$a_1$ to $a_{20}$ are preferably a hydrogen atom, a halogen atom, an alkyl group which may be substituted or an alkoxy group which may be substituted. One or more of $a_1$ to $a_5$, $a_6$ to $a_{10}$, $a_{11}$ to $a_{15}$ and $a_{16}$ to $a_{20}$ respectively is an alkoxy group having 4 to 20 carbon atoms which may be substituted, whereas the substitution position of the alkoxy group is preferably $a_2$ to $a_4$, $a_7$ to $a_9$, $a_{12}$ to $a_{14}$ and $a_{17}$ to $a_{19}$.

More preferred examples of combination include three combinations such as {$a_1$, $a_2$, $a_5$, $a_6$, $a_7$, $a_{10}$, $a_{11}$, $a_{12}$,

$a_{15}$, $a_{16}$, $a_{17}$ and $a_{20}$ are a hydrogen atom, while $a_3$, $a_4$, $a_8$, $a_9$, $a_{13}$, $a_{14}$, $a_{18}$ and $a_{19}$ are an alkoxy group having 4 to 20 carbon atoms which may be substituted}, {$a_1$, $a_3$, $a_5$, $a_6$, $a_8$, $a_{10}$, $a_{11}$, $a_{13}$, $a_{15}$, $a_{16}$, $a_{18}$ and $a_{20}$ are a hydrogen atom, while $a_2$, $a_4$, $a_7$, $a_9$, $a_{12}$, $a_{14}$, $a_{17}$ and $a_{19}$ are an alkoxy group having 4 to 20 carbon atoms which may be substituted} and {$a_1$, $a_2$, $a_4$, $a_5$, $a_6$, $a_7$, $a_9$, $a_{10}$, $a_{11}$, $a_{12}$, $a_{14}$, $a_{15}$, $a_{16}$, $a_{17}$, $a_{19}$ and $a_{20}$ are a hydrogen atom, while $a_3$, $a_8$, $a_{13}$ and $a_{18}$ are an alkoxy group having 4 to 20 carbon atoms which may be substituted}.

Further preferred examples thereof include the combination of {$a_1$, $a_2$, $a_4$, $a_5$, $a_6$, $a_7$, $a_9$, $a_{10}$, $a_{11}$, $a_{12}$, $a_{14}$, $a_{15}$, $a_{16}$, $a_{17}$, $a_{19}$ and $a_{20}$ are a hydrogen atom, while $a_3$, $a_8$, $a_{13}$ and $a_{18}$ are an alkoxy group having 4 to 20 carbon atoms which may be substituted}.

Here, the alkoxy group having 4 to 20 carbon atoms which may be substituted preferably has 5 to 20 carbon atoms and more preferably 5 to 12 carbon atoms from the viewpoint of solubility. When the number of carbon atoms is excessively low, the solubility might be insufficient. When the number is excessively high, the wet heat resistance might be worsened. So it is important to use a group having the proper number of carbon atoms.

Examples of the compound represented by the above formula (1-1) are shown in Tables 1-1 to 1-3. However, the present invention is not restricted thereto.

[Chemical Formula 5]

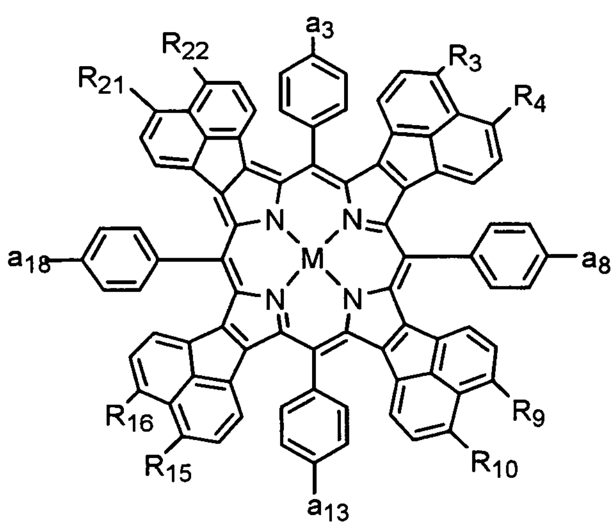

(1－2)

Table 1-1(1)

| No. | M | R3 | R4 | R9 | R10 | R15 | R16 |
|---|---|---|---|---|---|---|---|
| 1-1 | Cu | H | H | H | H | H | H |
| 1-2 | Cu | H | H | H | H | H | H |
| 1-3 | Cu | H | H | H | H | H | H |
| 1-4 | Cu | H | H | H | H | H | H |
| 1-5 | Pd | Cl | Cl | Cl | Cl | Cl | Cl |
| 1-6 | Ni | H | H | H | H | H | H |
| 1-7 | Ni | Cl | Cl | Cl | Cl | Cl | Cl |
| 1-8 | Co | Br | H | Br | H | Br | H |
| 1-9 | VO | CH3 | H | CH3 | H | CH3 | H |
| 1-10 | Zn | NO2 | H | NO2 | H | NO2 | H |

(continued)

| No. | M | R3 | R4 | R9 | R10 | R15 | R16 |
|---|---|---|---|---|---|---|---|
| 1-11 | Pt | Br | Br | Br | Br | Br | Br |
| 1-12 | Ni | $C_4H_9$ (n) | H | $C_4H_9$ (n) | H | $C_4H_9$ (n) | H |
| 1-13 | Co | $C_6H_{13}$ (n) | $C_6H_{13}$ (n) | $C_6H_{13}$ (n) | $C_6H_{13}$ (n) | $C_6H_{13}$ (n) | $C_6H_{13}$ (n) |

Table 1-1(2)

| No. | R21 | R22 | a3 | a8 | a13 | a18 |
|---|---|---|---|---|---|---|
| 1-1 | H | H | $OC_4H_9$ (n) | $OC_4H_9$ (n) | $OC_4H_9$ (n) | $OC_4H_9$ (n) |
| 1-2 | H | H | $OC_5H_{11}$ (n) | $OC_5H_{11}$ (n) | $OC_5H_{11}$ (n) | $OC_5H_{11}$ (n) |
| 1-3 | H | H | $OC_6H_{13}$ (n) | $OC_6H_{13}$ (n) | $OC_6H_{13}$ (n) | $OC_6H_{13}$ (n) |
| 1-4 | H | H | $OC_{10}H_{21}$ (n) | $OC_{10}H_{21}$ (n) | $OC_{10}H_{21}$ (n) | $OC_{10}H_{21}$ (n) |
| 1-5 | Cl | Cl | $OC_8H_{17}$ (n) | $OC_8H_{17}$ (n) | $OC_8H_{17}$ (n) | $OC_8H_{17}$ (n) |
| 1-6 | H | H | $OC_{10}H_{21}$ (n) | $OC_{10}H_{21}$ (n) | $OC_{10}H_{21}$ (n) | $OC_{10}H_{21}$ (n) |
| 1-7 | Cl | Cl | $OC_{10}H_{21}$ (n) | $OC_{10}H_{21}$ (n) | $OC_{10}H_{21}$ (n) | $OC_{10}H_{21}$ (n) |
| 1-8 | Br | H | $OC_6H_{13}$ (n) | $OC_6H_{13}$ (n) | $OC_6H_{13}$ (n) | $OC_6H_{13}$ (n) |
| 1-9 | CH3 | H | $OC_6H_{13}$ (n) | $OC_6H_{13}$ (n) | $OC_6H_{13}$ (n) | $OC_6H_{13}$ (n) |
| 1-10 | NO2 | H | $OC_5H_{11}$ (n) | $OC_5H_{11}$ (n) | $OC_5H_{11}$ (n) | $OC_5H_{11}$ (n) |
| 1-11 | Br | Br | $OC_7H_{15}$ (n) | $OC_7H_{15}$ (n) | $OC_7H_{15}$ (n) | $OC_7H_{15}$ (n) |
| 1-12 | $C_4H_9$ (n) | H | $OC_8H_{17}$ (n) | $OC_8H_{17}$ (n) | $OC_8H_{17}$ (n) | $OC_8H_{17}$ (n) |
| 1-13 | $C_6H_{13}$ (n) | $C_6H_{13}$ (n) | $OC_8H_{17}$ (n) | $OC_8H_{17}$ (n) | $OC_8H_{17}$ (n) | $OC_8H_{17}$ (n) |

[Chemical Formula 6]

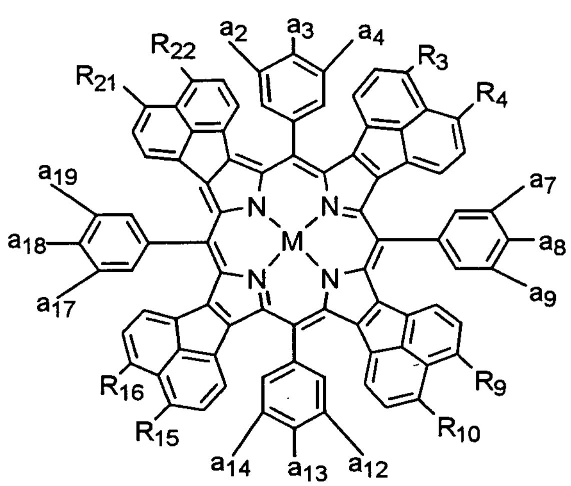

(1 − 3)

[Table 2]

Table 1-2

| No. | M | R3, R4, R9, R10, R15, R16, R21, R22 | a2, a3, a7, a8, a12, a13, a17, a18 | a4, a9, a14, a19 |
|---|---|---|---|---|
| 1-14 | Pd | H | $OC_8H_{17}$ (n) | H |
| 1-15 | Cu | C2H5 | $OC_4H_9$ (n) | H |
| 1-16 | VO | OCH3 | $OC_{12}H_{23}$ (n) | H |
| 1-17 | Zn | Cl | $OC_{10}H_{21}$ (n) | H |

[Table 3]

Table 1-3

| No. | M | R3, R4, R9, R10, R15, R16, R21, R22 | a2, a4, a7, a9, a12, a14, a17, a19 | a3, a8, a13, a18 |
|---|---|---|---|---|
| 1-18 | Cu | H | $OC_6H_{13}$ (n) | H |
| 1-19 | Ni | Br | $OC_4H_9$ (n) | H |
| 1-20 | Co | Cl | $OC_5H_{11}$ (n) | H |
| 1-21 | Sn | C3H7 (n) | $OC_{12}H_{23}$ (n) | H |

In the compound represented by the above formula (2-1), $R_1$ to $R_{24}$ have each the same meanings as those in $R_1$ to $R_{24}$ in the above formula (3-1) and its preferable range is also the same. Further, M has the same meanings as those in M in the above formula (3-1) and its preferable range is also the same.
In the above formula (2-1), $B_1$ to $B_4$ each independently represent an alkenyl group which may be substituted, an alkynyl group which may be substituted or a heteroaryl group which may be substituted. Since the substituents of $B_1$ to $B_4$ have an influence on absorption wavelength of a dye, a substituent is preferably selected depending on the desired absorption wavelength. Concrete examples of the substituent are the same as those described above.

In the compound represented by the above formula (2-2), M has the same meanings as those in M in the above formula (3-1) and its preferable range is also the same.
Further, $Q_1$ to $Q_{24}$ have each the same meanings as $R_1$ to $R_{24}$ in the above formula (3-1). $Q_1$ to $Q_{24}$ are preferably a hydrogen atom, a halogen atom, a nitro group, an alkyl group which may be substituted, an aryl group which may be substituted, an alkoxy group which may be substituted or an aryloxy group which may be substituted, form the viewpoints of solubility, absorption wavelength, sharpness of absorption peak and easiness of production of the above compound. Concrete examples of each substituent in the above are the same as those described in the compound represented by the above general formula (3-1).

However, one or more of $Q_1$ to $Q_{24}$ are a nitro group, a cyano group, a hydroxyl group, a carboxyl group, a sulfonic acid group, an alkoxycarbonyl group which may be substituted, an aryloxycarbonyl group which may be substituted, an aminocarbonyl group which may be substituted, an amino group which may be substituted, an aminosulfonyl group which may be substituted, an alkynyl group which may be substituted or a heteroaryl group which may be substituted. However, one or more of the substituents which must be taken are preferably a nitro group, a cyano group, a hydroxyl group or a heteroaryl group which may be substituted, and more preferably a nitro group.

In $Q_1$ to $Q_{24}$, $Q_1$ to $Q_2$, $Q_5$ to $Q_8$, $Q_{11}$ to $Q_{14}$, $Q_{17}$ to $Q_{20}$ and $Q_{23}$ to $Q_{24}$ are more preferably a hydrogen atom, while $Q_3$ to $Q_4$, $Q_9$ to $Q_{10}$, $Q_{15}$ to $Q_{16}$ and $Q_{21}$ to $Q_{22}$ are a hydrogen atom, a halogen atom, a nitro group, an alkyl group which may be substituted or an alkoxy group which may be substituted. However, in $Q_3$ to $Q_4$, $Q_9$ to $Q_{10}$, $Q_{15}$ to $Q_{16}$ and $Q_{21}$ to $Q_{22}$, one or more of the substituents which must be taken is preferably a nitro group.

In the compound represented by the above general formula (2-2), $A_5$ to $A_8$ each independently represent a hydrogen atom, an alkyl group which may be substituted or an aryl group which may be substituted. Since the substituent of $A_5$ to $A_8$ have an influence on absorption wavelength of a dye, a substituent is preferably selected depending on the desired absorption wavelength. Concrete examples of the substituent are the same as those described above.
Examples of the compound represented by the above formulae (2-1) and (2-2) are shown in Tables 2-1 to 2-3. However, the present invention is not restricted thereto.

[Chemical Formula 7]

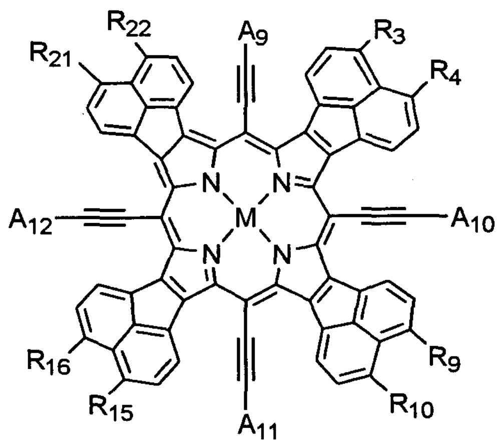

(2 − 3)

[Table 4]

Table 2-1

| No. | M | R3, R9, R15, R21 | R4, R10, R16, R22 | A9 to A12 |
|---|---|---|---|---|
| 2-1 | Pd | CH3 | H | SiMe3 |
| 2-2 | VO | Cl | Cl | Ph |
| 2-3 | Co | Br | H | Ph |
| 2-4 | Fe | Br | Br | $C_5H_{11}$ (n) |
| 2-5 | Cu | H | H | 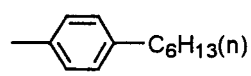 |
| 2-6 | Cu | C2H5 | C2H5 | 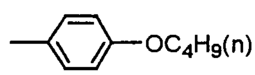 |
| 2-7 | Ni | OCH3 | OCH3 | Ph |
| 2-8 | Zn | NO2 | H | SiMe3 |
| 2-9 | Pd | OH | OH | $C_5H_{11}$ (n) |

[Chemical Formula 8]

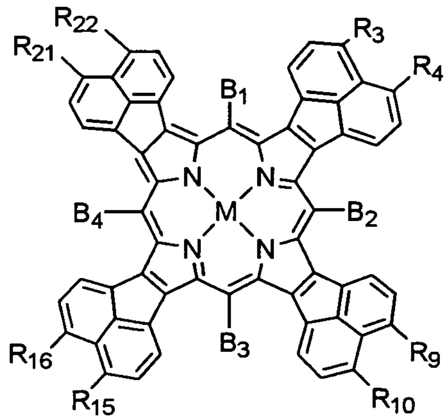

(2 - 4)

[Table 5]

Table 2-2

| No. | M | R3, R9, R15, R21 R3, R9, R15, R21 | R4, R10, R16, R22 R4, R10, R16, R22 | B1 to B4 B1 to B4 |
|---|---|---|---|---|
| 2-10 | Co | H | H | 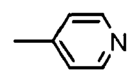 |
| 2-11 | Pd | Cl | Cl | 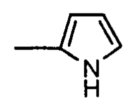 |
| 2-12 | Cu | H | H | 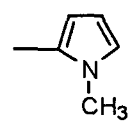 |
| 2-13 | Ni | H | H | 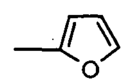 |
| 2-14 | Ni | OC2H5 | OC2H5 | 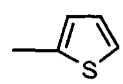 |
| 2-15 | Pt | CH3 | CH3 | 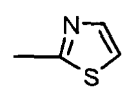 |
| 2-16 | Cu | H | H | 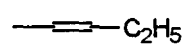 |
| 2-17 | Pd | NO2 | H | 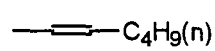 |
| 2-18 | Ni | NMe2 | H | 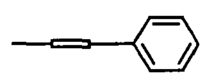 |

[Chemical Formula 9]

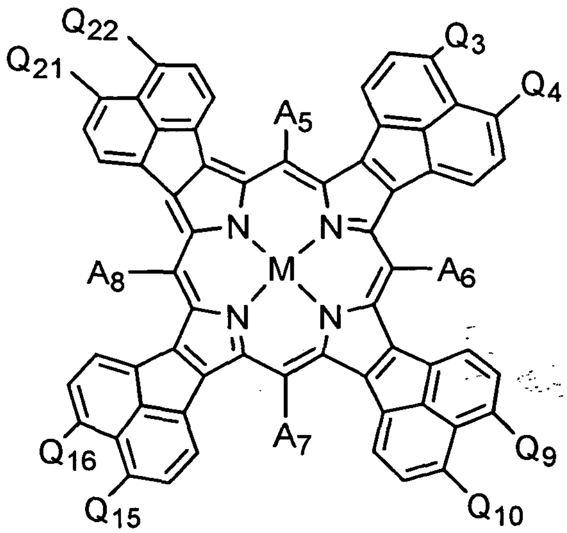

(2－5)

[Table 6]

Table 2-3

| No. | M | Q3, Q9, Q15, Q21 | Q4, Q10, Q16, Q22 | A5 to A8 |
|---|---|---|---|---|
| 2-19 | Co | NO2 | H | H |
| 2-20 | Pd | CN | H | Ph |
| 2-21 | Zn | NH2 | H | CH3 |
| 2-22 | Ni | NHCOCH3 | H | C2H5 |
| 2-23 | Pt | OH | H | $C_4H_9$(iso) |
| 2-24 | Cu | CONH2 | H | $C_6H_{13}$(n) |
| 2-25 | Pd | OH | OH | 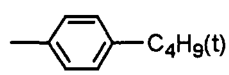 |
| 2-26 | Ni | SO2NH2 | H | 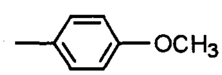 |

The aforementioned tetra-acenaphthoporphyrin compound can be produced in accordance with a known method as described, for example, in J. Am. Chem. Soc. , Vol. 118, pp. 8767 to 8768 (1996) and the like. For example, acenaphthopyrrole and benzaldehyde (As needed, alkoxybenzaldehyde or the like may also be used.) are condensed by using Lewis acids such as a boron trifluoride/ethyl ether complex, trifluoroacetic acid or the like as a catalyst, which is oxidized with 2,3-dichloro-5,6-dicyano-1,4-benzoquinone, to obtain a tetra-acenaphthoporphyrin compound in which M is a hydrogen atom. When the resulting compound is heated along with acetate of a metal, a porphyrin compound in which M is a metal is obtained.

The compound represented by the above formulae (1-1), (2-1) and (2-2) is excellent in light resistance, heat resistance and wet heat resistance, is good at solubility, and has a selective absorption. For this reason, the filter containing the above compound is useful as an optical filter for display such as a liquid crystal display (LCD), a plasma display panel (PDP), an electroluminescence display (ELD), a cathode ray tube (CRT), a fluorescent display tube and a field emission display, and particularly the most suitable as an optical filter for PDP. Furthermore, in addition thereto, such a compound can also be used as inkjet ink, various printing inks, a heat sensitive recording material, a pressure sensitive recording material, electrophotography, a color toner, a color filter, an optical recording medium, a photosensitizer, a solar cell and the like. Further, it may be contained in a resin in a melted or dispersed state and used as a

coloring agent.

The optical filter of the present invention preferably has a percentage of light transmittance at a wavelength of 550 nm to light transmittance at a wavelength of 525 nm of not more than 85%, more preferably not more than 80%, further preferably not more than 75%. When the percentage of light transmittances is more than 85%, a necessary emission inherent in a display unit is also decreased or the efficiency of eliminating an unnecessary light existing in the vicinity of 550 nm is worsened so that the contrast tends not to be improved so much and the luminance tends to be remarkably decreased as well.

Light in the vicinity of 550 nm refers to light having very strong luminous efficiency and light of a wavelength contained in external light such as a fluorescent lamp or the like. When light in the vicinity of 550 nm is visualized, an image with enhanced luminance of black image is given. Such an image becomes an image with lowered contrast, as a result. If external light is not visualized whenever possible, excellent contrast can be realized without losing the contrast inherent in a display unit due to external light. As light recognized by a viewer, there is light (b) which is external light from a fluorescent lamp or the like reflected on a surface of a display unit or inside a display unit in a bright place, in addition to light (a) mainly emitted from a light source inside a display unit. This (b) is visualized, whereby luminance of black image is increased, and as a result, an image with lost contrast inherent in a display unit is given. For this reason, if a method to reduce only the unnecessary external light between a position on which external light is reflected and a visible area is found out, it is possible to achieve a display unit having excellent contrast, and particularly excellent contrast in a bright place, without losing the original contrast due to external light. All known methods can be used as far as the above effect is obtained. However, if external light alone can be eliminated by a filter to be arranged in a front surface of a display, such a method is more convenient and efficient. Namely, if light in the vicinity of 550 nm alone is reduced without remarkably decreasing emission of a display by an optical filter to be arranged in a front surface of a display, it is possible to achieve a display having excellent contrast, and particularly excellent contrast in a bright place.

Furthermore, light in a wavelength of from 530 to 570 nm can be an unnecessary light for decreasing the color purity of green color in case of emission of a display. When emission of not more than 530 nm is absorbed, green color emission of a display is also decreased in some cases. The optical filter of the present invention eliminates only an unnecessary light in the wavelength region without greatly decreasing light transmittance of a necessary light as green color emission of a display, whereby the color purity (particularly, the color purity of green color) of a display can also be enhanced. The light transmittance in the present invention refers to a percentage of the tristimulus value Y specified in JIS Z8724. Light transmittance is measured according to a method as specified in JIS R3106.

Here, the external lights include mainly a three-band fluorescent tube. The three-band fluorescent tube refers to a light source in which the wavelength is mainly concentrated on three spectral bands (435 nm, 545 nm and 610 nm) . Typical examples thereof include three-band daylight color, three-band white light color, three-band white color, three-band warm white color, three-band warm white color and the like.

Meanwhile, the optical filter of the present invention is composed of a plurality of members such as a multi-layer structure to be described later. A dye to be used does not need to be contained in a member alone, but it may be contained in a plurality of members.

The optical filter in the present invention more preferably contains a dye (A) having an absorption maximum in a wavelength range of from 440 to 510 nm, and/or a dye (B) having an absorption maximum in a wavelength range of from 575 to 620 nm.

The dye (A) to be used in the present invention is not particularly limited as far as it is a compound having a desired absorption band in the above wavelength range. Concrete examples thereof include an anthraquinone type compound, a phthalocyanine type compound, a methine type compound, an azomethine type, an oxazine type compound, an azo type compound, a styryl type compound, a porphyrin type compound, a pyrromethene type compound, a squarylium type compound, a cyanine type compound, an oxonol type compound and the like. The dye (A) may also be used in combination of two or more kinds thereof. Furthermore, the dye (A) may be contained in a plurality of members as well.

The dye (B) to be used in the present invention is not particularly limited as far as it is a compound having a desired absorption band in the above wavelength range. Concrete examples thereof include all dyes as described in the dye (A). The dye (B) may also be used in combination of two or more kinds thereof. Furthermore, the dye (B) may be contained in a plurality of members as well.

Furthermore, according to the desired optical properties, a dye having an absorption band in a visible light region or a dye having an absorption band in a near-infrared ray region may be used together, in addition to the aforementioned tetra-acenaphthoporphyrin compound, dye (A) and dye (B) for adjusting the optical properties.

As the member constituting the optical filter of the present invention, conventionally known members can be used without restriction. Examples thereof include a transparent base material (C), an electrically conductive layer (D) and a functional transparent layer (E). Furthermore, as the member constituting the optical filter of the present invention, an adhesive material layer, a binding material layer, an impact relaxing layer, an electrode and the like may be used.

The optical filter of the present invention preferably has an electrically conductive layer (D) and/or a functional transparent layer (E). The optical filter of the present invention more preferably has a toning layer (F). When the optical filter is

provided with the transparent base material (C), the electrically conductive layer (D) and the functional transparent layer (E), it preferably has a lamination structure comprising at least the transparent base material (C) / the electrically conductive layer (D) / the functional transparent layer (E) in that order. The optical filter more preferably has such a lamination structure and the toning layer (F) at the same time.

Examples of the electrically conductive layer (D) include a transparent thin film of a metal, a metal oxide or the like, a metal mesh layer and the like. Furthermore, examples of the functional transparent layer (E) include known functional transparent layers such as an anti-reflective layer, an anti-glare layer, an anti-fouling layer, a hard coat layer, an anti-Newton ring layer and the like. One layer may have two or more functions as described above.

In the optical filter of the present invention, the aforementioned tetra-acenaphthoporphyrin compound is preferably contained in any one of the above layers. The toning layer (F) preferably contains a dye and more preferably contains the above tetra-acenaphthoporphyrin compound, dye (A) and dye (B). However, it is not restricted thereto. Furthermore, the optical filter having a constitution containing the above tetra-acenaphthoporphyrin compound and preferably the dye (A) and/or dye (B) in the member of the above respective layers except the toning layer (F) is also included in the present invention.

The constitutions of the optical filter of the present invention, for example, in case of an optical filter for a plasma display, include (i) a construction comprising at least a transparent base material (C), an electrically conductive layer (D) and a functional transparent layer (E), and the dye of the present invention contained in at least one member constituting the transparent base material (C), the electrically conductive layer (D) and the functional transparent layer (E), and (ii) a construction comprising a toning layer (F) containing the dye of the present invention, in addition to at least a transparent base material (C), an electrically conductive layer (D) and a functional transparent layer (E) as its preferred embodiment. The optical filter for a plasma display in the present invention is not restricted to the above (i) and (ii).

A method for supporting the dye in the member is not particularly limited, but concrete examples thereof include (1) a method for allowing the dye to be contained in the transparent base material (C) such as a polymeric molded article or the like, (2) a method for applying the dye on a surface of the transparent base material (C) as a hard coat layer, (3) a method for allowing the dye to be contained in an adhesive and the like. As the method for allowing the dye to be contained, all known techniques may be used as far as they can disperse the dye uniformly.

Concrete examples of the method (1) for allowing the dye to be contained in the polymeric molded article include a method which usually comprises adding the dye in a powder or pellet of a base resin, heat-melting and preferably kneading the mixture at 150 ˚C to 350 ˚C while considering the melting temperature of the resin, and then molding it to prepare a plastic sheet and plastic particles, although the processing temperature and film-making conditions are different depending on the kind of the base resin to be combined with the dye, and the like. For the purpose of easy molding or the like, an additive such as a plasticizer or the like may be added thereto.

The other methods include a casting method. In the casting method, the dye is added and dissolved in a resin solution in which a resin or a resin monomer is dissolved in an organic solvent and, on this occasion, a plasticizer, a polymerization initiator or an anti-oxidant is added, as desired, and the resulting solution is poured onto a mold which has a required surface contour to obtain a polymeric molded article through subsequent solvent evaporation/drying or polymerization/solvent evaporation/drying processing.

Although the amount of the dye added is different depending on the absorption coefficient of the dye, the thickness of the polymeric molded article and the desired transmission characteristics, it is usually from 1 (wt)ppm to 20 (wt)% based on the base resin molded article.

Specific examples of the method (2) for casting the dye on the surface of the polymeric molded article or glass as the hard coat layer include a method in which a coating composition prepared by dissolving the dye in a binder resin and an organic solvent or an acrylic emulsion water paint prepared by pulverizing the dye to a particle size of from 50 to 500 nm and dispersing in an uncolored acrylic emulsion coating material is coated and dried according to a conventionally known coating method such as a bar coater, a blade coater, a spray coater, a reverse coater, a die coater, spray or the like.

Although the concentration of the dye is different depending on the absorption coefficient of the dye, the thickness of the coating and the desired optical characteristics, it is usually from 1 (wt)ppm to 30 (wt)% based on the binder resin. An anti-oxidant or the like may be added in the coating composition. In order to protect the coated surface, a protective layer may be formed on the coated surface.

Next, specific examples of the method (3) for allowing the dye to be contained in the adhesive will be illustrated. In the present invention, an adhesive material, a cohesive agent, a cohesive agent, an adhesive agent and an adhesive material are described as the adhesive without distinction. Examples of the adhesive include those of an acrylic resin, a silicone resin, a urethane resin, a polyvinyl butyral resin, an ethylene-vinyl acetate resin, a polyvinyl ether resin, saturated amorphous polyester, a melamine resin and the like in sheet form or in liquid form. A dye-containing adhesive is prepared by adding the dye to the adhesive.

Although the amount of the dye added is different depending on the absorption coefficient of the dye, the thickness of the adhesive and the desired optical characteristics, it is usually from 1 (wt)ppm to 30 (wt)% based on the base adhesive.

Examples of the method for forming the adhesive include a method which comprises directly coating the adhesive to a substrate to form the adhesive or a method which comprises peeling away a mold releasing film on one surface of the adhesive sandwiched between mold releasing films and attaching it to a substrate, but in the present invention, the latter method is properly used. Examples of the method for coating the adhesive generally include a reverse coating method, a bar coating method, a gravure coating method, a roll coating method and the like. However, the method is not restricted thereto. In the present invention, a bar coating method can be properly used. The thickness of the adhesive is not particularly limited, but it is from 0.5 to 50 μm and preferably from 1 to 30 μm.

The adhesive containing the dye is also used for various attachment of film and film, film and glass and the like, resulting in decreasing the number of members. For this reason, the above method (3) is one of preferred embodiments as a method for incorporating the dye.

The optical filter suited for a display can increase its temperature because it is often the case that the display panel has a high surface temperature and the display is used in a hot environment. For this reason, when the dye of the present invention is contained in the optical filter to be properly used for the purpose of a display, it is preferable that the dye has heat resistance which does not cause remarkable deterioration, for example, due to decomposition at 80 °C or the like.

Specifically, the heat resistance of the dye used in the present invention is preferably such that the absorption retention rate of the maximum absorption wavelength of the dye is not less than 80% after the dye is held at 80 °C for 250 hours, preferably not less than 80% after the dye is held for 500 hours or more, and more preferably not less than 80% after the dye is held for 1000 hours or more.

The absorption retention rate is represented by the following equation (2):

$$\text{Absorption retention rate (\%)} = (T_0 - T_{max,1})/(T_0 - T_{max,0}) \times 100 \quad (2)$$

The absorption retention rate is calculated based on the difference ($T_0$-$T_{max,0}$) wherein $T_0$ is light transmittance at a wavelength where the dye does not absorb the light and $T_{max,0}$ is light transmittance at a maximum absorption wavelength of the dye, and refers to a change between the above difference and a difference between To and light transmittance $T_{max,1}$ at a maximum absorption wavelength after the dye is exposed to each environment for a predetermined time. When there are a plurality of absorption maxima, $T_{max,0}$ is light transmittance at a maximum absorption wavelength showing the highest absorption coefficient. $T_{max,1}$ represents light transmittance at the same wavelength as that in $T_{max,0}$ or at a maximum absorption wavelength nearest to the wavelength in $T_{max,0}$. °C.

Here, light transmittance To at a wavelength where the dye does not absorb the light represents an average light transmittance (%) of light transmittances at from 380 to 780 nm that are constant in a wavelength range of 30 nm. The constant light transmittance refers to the deviation of the light transmittance of within ±1%. When there are a plurality of wavelength ranges in which light transmittance is constant in a wavelength range of 30 nm, $T_0$ is an average value (%) of light transmittance in a wavelength range with the highest light transmittance.

To evaluate heat resistance of the dye, a sample prepared in the follow manner was used.

A dye-containing diluting agent was prepared such that the dye concentration in the resin was 1000 (wt)ppm when the dye was dispersed and dissolved in a solvent and dried. Subsequently, an acrylic adhesive (a copolymer of butyl acrylate and acrylic acid (Product name: DBBOND, manufactured by Diabond Industry Co., Ltd.) and the dye-containing diluting agent are mixed (weight ratio 80:20), and continuously applied on a mold releasing layer of a mold releasing film comprising a polyethylene terephthalate film and a silicone mold releasing layer according to the bar coating method, and the resulting material was dried. Due to this, an acrylic adhesive containing the dye having a film thickness of 25 μm was prepared. The prepared adhesive was attached to a transparent acrylic resin (manufactured by Mitsubishi Rayon Co., Ltd., Acrylite, thickness: 2mm), and the mold releasing film of the adhesive was peeled away and an ultraviolet blocking film (manufactured by Teijin Co., Ltd., HB Film, thickness: 75 μm) was attached to give the sample for evaluation.

The thus obtained sample was kept under an environment of 80 °C for a predetermined time to obtain an absorption retention rate represented by the above equation (2).

The higher the value of this absorption retention rate is, the dye maintains its performance, that is, the more durable the dye is.

Further, there are some dyes which are deficient in light resistance as well as heat resistance and there might be a problem of deterioration in the dye due to emission of the display itself or an ultraviolet ray and/or a visible light ray contained in external light. In this case, the deterioration of the dye may be reduced by known techniques for selectively blocking light. For example, a member (layer) which contains an ultraviolet absorbent or which is capable of blocking ultraviolet light, or a member which contains a dye that absorbs visible light of a wavelength causing deterioration of the dye or which is capable of blocking the visible light may be provided between the layer containing the dye and the

incident source of the ultraviolet light and visible light. Of course, if a dye which is not deteriorated due to an ultraviolet ray and/or a visible light ray can be adopted, the above technique does not need to be used together.

As the preferred light resistance of the dye to be used in the present invention, the absorption retention rate represented by the following equation (1) is not less than 80% when a visible light ray of from 380 to 780 nm is irradiated at 50 mW/cm$^2$ for 250 hours, preferably not more than 80% when irradiated for 500 hours, and more preferably not less than 80% when irradiated for 1000 hours.

$$\text{Absorption retention rate (\%)} = (T_0 - T_{max,1}) / (T_0 - T_{max,0}) \times 100 \quad (1)$$

To evaluate light resistance of the dye, a sample prepared in the same method as in evaluation of heat resistance is used. The absorption retention rate represented by the above equation (1) is measured in the same manner as in heat resistance. However, in this case, the obtained sample is not kept under the environment of 80 °C, but the above visible light ray is irradiated on a side of the ultraviolet blocking film of the sample in a room at a temperature of 30 ± 10 °C, a humidity of 50 ± 20% for a predetermined time. $T_{max,1}$ represents light transmittance in a maximum absorption wavelength after the above visible light ray is irradiated for a predetermined time. When there are a plurality of absorption maxima, $T_{max,0}$ is light transmittance at a maximum absorption wavelength showing the highest absorption coefficient. $T_{max,1}$ represents light transmittance at the same wavelength as that in $T_{max,0}$ or at a maximum absorption wavelength nearest to the wavelength in $T_{max,0}$.

Furthermore, the present invention relates to an optical filter containing a dye which has an absorption maximum at a wavelength range of from 530 to 570 nm and the ratio of the absorption coefficient at a wavelength of 550 nm to the absorption coefficient in a wavelength of 525 nm of not less than 1.2, and which has an absorption retention rate represented by the following equation (1) of not less than 80% when a visible light ray in a wavelength of from 380 to 780 nm is irradiated at 50 mW/cm$^2$ for 250 hours on an acrylic adhesive containing the dye.

$$\text{Absorption retention rate (\%)} = (T_0 - T_{max,1}) / (T_0 - T_{max,0}) \times 100 \quad (1)$$

Evaluation of light resistance of the above dye is also carried out in the same manner as in evaluation of the above light resistance, while the absorption retention rate represented by the above equation (1) is measured in the same manner as in the above absorption retention rate as well.

The absorption retention rate represented by the above equation (1) is not less than 80%, preferably not less than 80% when irradiating for 500 hours, and more preferably not less than 80% when irradiating 1000 hours.

The dyes satisfying this condition are not particularly limited, but include the above tetra-acenaphthoporphyrin compound.

Similarly, under the environment of humidity, in addition to heat and light, and combined environment thereof, a dye having high absorption retention rate is preferable. When the dye is deteriorated, transmission characteristics of the filter for the display itself are changed and the color tone is changed in some cases. Further, the near-infrared blocking capacity and the like are also changed in some cases.

The above tetra-acenaphthoporphyrin compound is highly excellent in environmental resistance such as the above heat resistance, light resistance and the like, as compared to existing dyes, and particularly a dye with a maximum absorption wavelength existing in a wavelength of from 530 to 570 nm, and can be used without deteriorating performance over a long period of time. In general, durability of a dye in an adhesive is inferior to that of a coated film or the like. However, since the above tetra-acenaphthoporphyrin compound has very high durability even in an adhesive, it can be properly used for an optical filter.

Furthermore, as characteristics required for the dye, solubility or dispersion into a solvent is also important in order to disperse the dye in a medium or a coated film. The above tetra-acenaphthoporphyrin compound can provide sufficient solubility or dispersion if the structure of each dye is controlled according to the application.

By the optical filter of the present invention containing the above dye, the color purity of all known displays can be enhanced. In particular, the optical filter can be properly used for a plasma display, a liquid crystal display, and an organic electroluminescence display. Namely, the plasma display according to the present invention is provided with the optical

filter as described above, and the liquid crystal display and the organic electroluminescence display according to the present invention are provided with the optical filter as described above. As a method of application, all known methods can be adopted.

Each member which can be used for the above optical filter will be briefly described below.

As a transparent base material (C), preferred is a base material which is mainly in a film form and a plate form, excellent in transparency, and has a mechanical strength according to the application. Here, being excellent in transparency refers to a visible light transmittance of not less than 40% in a thickness in a state of being used (measurement method: JIS R-3106). Examples thereof include a polymer film, a polymer plate or a glass plate. In the present invention, the known transparent base material as described above can be used without restriction.

The above electrically conductive layer (D) preferably has at least an electromagnetic wave shielding function. This is attributable to the fact that a display unit such as a plasma display panel or the like generates leakage electromagnetic wave and/or near-infrared ray based on its structure and operating principles, and there is a need to shield them. In order to shield such an electromagnetic wave, a surface of the display is usually covered with an electrically conductive substance having high electric conductivity in many cases. This is due to a mechanism exhibiting the electromagnetic wave shielding capacity in which the above electrically conductive substance absorbs an electromagnetic wave for escaping it as electric charge. In order to apply this principle to the optical filter of the present invention, the electrically conductive layer (D) is preferably transparent with respect to a visible light ray. For this reason, concrete preferred examples of the electrically conductive layer (D) include a single-layered metal thin film, an oxide semiconductor film, a transparent electrically conductive thin film of a multi-layered thin film, an electrically conductive mesh, a synthetic fiber which has been coated with a metal, a metallic fiber mesh or a metallic fiber mesh which has further been coated with a metal and the like. The electric conductivity of the electrically conductive layer (D) necessary for shielding the electromagnetic wave as various display units such as a plasma display and the like also depends on required electromagnetic wave standard or radiation intensity from a plasma display. However, a surface resistance thereof is preferably not more than 10 Ω/square and more preferably 3 Ω/square. In case of strict electromagnetic wave standard required for consumer use and the like, a surface resistance thereof is preferably not more than 1 Ω/square and more preferably not more than 0.1 Ω/square. Further, in order to shield a near-infrared ray emitted from a plasma display, transmittance of the optical filter in a near-infrared ray wavelength range of from 800 to 1000 nm is preferably not more than 20%.

As the electrically conductive layer (D) in the present invention, a multi-layered transparent electrically conductive thin film layer (a) with a metal thin film and a transparent high refractive index-thin film laminated thereto, or an electrically conductive mesh layer (b) can be properly used. The multi-layered transparent electrically conductive thin film layer (a) is cheaper than the electrically conductive mesh layer, and combines an electromagnetic wave shielding function and a near-infrared ray shielding function by its property of reflecting a near-infrared ray due to free electrons of a metal constituting the metal thin film. On the other hand, the electrically conductive mesh layer (b) can have much higher electric conductivity without remarkably deteriorating the optical characteristics. For this reason, when a high electromagnetic wave shielding function is required, for example, it is preferable that the above strict electromagnetic wave standard for consumer use or the like can be clear.

Preferably, the multi-layered transparent electrically conductive thin film layer (a) is constituted by a laminate including 1 to 6 units each consisting of a high refractive-index layer (c) based on a dielectric material such as indium oxide, zinc oxide or titanium oxide and a metal thin film layer (d) based on silver ((c)/(d)), and the laminate is provided on one surface of the transparent base material (C). Furthermore, it is more preferable that all metal thin film layers (d) are laminated such that they are sandwiched between high refractive-index layers (c). The higher the number of lamination of the high refractive-index layer (c) and the metal thin film layer (d) is, a balance between the electric conductivity and transparency is desirable. In consideration of the cost and quality stability, however, the number of lamination is preferably not more than 11 layers, more preferably not more than 9 layers, and further preferably not more than 7 layers. Further, in order to enhance environmental resistance of the transparent electrically conductive thin film layer, a protective layer of an organic substance or an inorganic substance may be formed on a surface of the transparent electrically conductive thin film layer. Further, in order to enhance environmental resistance of the metal thin film layer and adhesion of the high refractive-index layer and the metal thin film, a functional layer may be formed between the high refractive-index layer and the metal thin film layer.

As the specific compounds constituting the above high refractive-index layer (c) and the metal thin film layer (d), known compounds are used without restriction. For example, compounds as described in Japanese Patent Publication No. 3004222 or the like are properly used. More concretely, preferred examples of the high refractive-index layer (c) include metal oxides such as indium oxide, indium-tin oxide (ITO), titanium oxide, zinc oxide, indium-cerium oxide (ICO) and the like. Furthermore, preferred examples of the metal thin film layer (d) include silver, or an alloy of silver with other metals such as gold, copper, palladium, platinum, neodymium, bismuth and the like. Further, as a method for forming these thin film layers, there may be employed any of all known methods, in addition to a vacuum thin film coating method such as a sputtering method, a vacuum deposition method, an ion plating method, an ion beam assisted deposition method and the like. Of these methods, preferred is a vacuum thin film coating method, and particularly

preferred is a sputtering method.

As the electrically conductive mesh layer (b), a fiber mesh in which a fiber has been deposited with a metal, an etching mesh which has been produced by first forming a pattern by using a photolithography technique to obtain a mesh by etching or the like can be used. More concretely, a metallic mesh mainly using copper is properly used. The shape of the mesh is not particularly limited, and it may be a lattice type or a honeycomb type. A high electromagnetic wave shielding function is obtained by using this mesh. When the electrically conductive mesh layer (b) is used as the electrically conductive layer (D), a layer for absorbing a near-infrared ray or reflecting a near-infrared ray is preferably used to impart a near-infrared ray shielding function. More preferably used is a layer which contains a dye for absorbing a near-infrared ray and hardly absorbs a visible light ray or never absorbs a visible light ray. Of course, the above transparent electrically conductive thin film layer may also be used.

Concrete examples of the function of the functional transparent layer (E) include at least one function selected from known performance and functions such as a hard coat property, an anti-reflection property, an anti-glare property, an antistatic property, an anti-fouling property, a gas barrier property, a near-infrared blocking property, an ultraviolet blocking property, a toning function, a mesh smoothing function and the like. The function to be imparted is not restricted thereto. In particular, a layer having an ultraviolet blocking capacity is preferably arranged in a human side. Once the functional transparent layer (E) has the above functions such as an anti-reflection property and the like, it may be of any types such as a membrane, a film, an adhesive layer and its complex or the like. A method for forming the functional transparent layer (E) is not particularly limited, and any known techniques can be used. Further, the functional transparent layer (E) may be made of two or more layers, or one layer thereof may have a plurality of functions.

More specifically, as these members, those as described, for example, in Japanese Patent Publication No. 3004222 and the like are suitably used. Further, as a method for laminating these layers, known methods can be used without restriction. More concretely, methods as described in Japanese Patent Publication No. 3004222 and Japanese Patent Laid-open No. 2002-40233 and the like can be adopted.

Since the plasma display panel provided with the optical filter obtained as described above can eliminate an unnecessary emission emitted from a light source without deteriorating performance over a long period of time, the color purity of the present invention, and particularly the color purity of blue color, is enhanced. Further, since the optical filter of the present invention can also eliminate an unnecessary light due to external light, a display with enhanced contrast even in a bright place can be provided.

In the liquid crystal display and the organic electroluminescence display provided with the optical filter of the present invention, the above optical filter is preferably placed between the light source and the visible area. The liquid crystal display will be described as an example below, whereas the same method can also be applied to the organic electroluminescence display.

As one of preferred embodiments for carrying out the optical filter of the above liquid crystal panel, at least the transparent base material (C), the functional transparent layer (E), and as needed, the toning layer (F) are provided, and the dye of the present invention is contained in at least one of constituent layers and constituent members.

As the transparent base material (C) and the functional transparent layer (E) of the optical filter of the above liquid crystal panel, the same materials as described in the above filter for the plasma display can be used. Further, as the method for laminating the above layers, conventionally known methods can be adopted without restriction. As preferred materials and methods above, materials and methods as described, for example, in Japanese Patent Laid-open No. 2002-40233 and the like can be adopted.

Suitable examples of the method for applying the optical filter of the present invention to the liquid crystal display include (I) a method which comprises attaching the optical filter to a constituent member of the liquid crystal display via an adhesive layer and (II) a method which comprises inserting it between the light source and the visible area of the liquid crystal display, prior to use. Further, the method for applying the optical filter of the present invention to the liquid crystal display is not restricted thereto.

The liquid crystal display or the organic electroluminescence display provided with the optical filter obtained as described above eliminates an unnecessary emission emitted from a light source without deteriorating performance over a long period of time in the same manner as in the aforementioned plasma display, so the contrast in a bright place is enhanced. Further, the color purity of luminescent color can be improved. A plurality of filters of the same kind may be used.

The optical filter of the present invention may be used in combination with known displays such as a CRT display, a field emission display, an inorganic electroluminescence display and the like, in addition to the above. Further, the above display provided with the optical filter of the present invention eliminates an unnecessary light without deteriorating performance over a long period of time, so it is possible to provide beautiful images as a display which is excellent in the contrast, and also excellent in the color purity of luminescent color.

## EXAMPLES

The present invention is now more specifically illustrated below with reference to Examples. However, the

present invention is not restricted to these Examples.

Example 1

Compound represented by the above formula (1-1) and optical filter using the compound

Example 1-1

In a nitrogen atmosphere, into a flask were introduced 5.2 g of p-decyloxybenzaldehyde and 1L of chloroform. Then, 4. 0 g of acenaphthopyrrole was added thereto and the resulting mixture was stirred. 0.55 g of $BF_3 \cdot Et_2O$ was added thereto and the mixture was stirred for 4 hours, and then 3.3 g of 2,3-dichloro-5,6-dicyano-1,4-benzoquinone was added and the mixture was stirred for 2 hours. The solvent was distilled off and then the residue was purified with a silica gel column. In the purification, chloroform (1% triethylamine contained) was used as a developing solution, whereby 7.8 g of a compound represented by the following formula (1-A) was obtained.

[Chemical Formula 10]

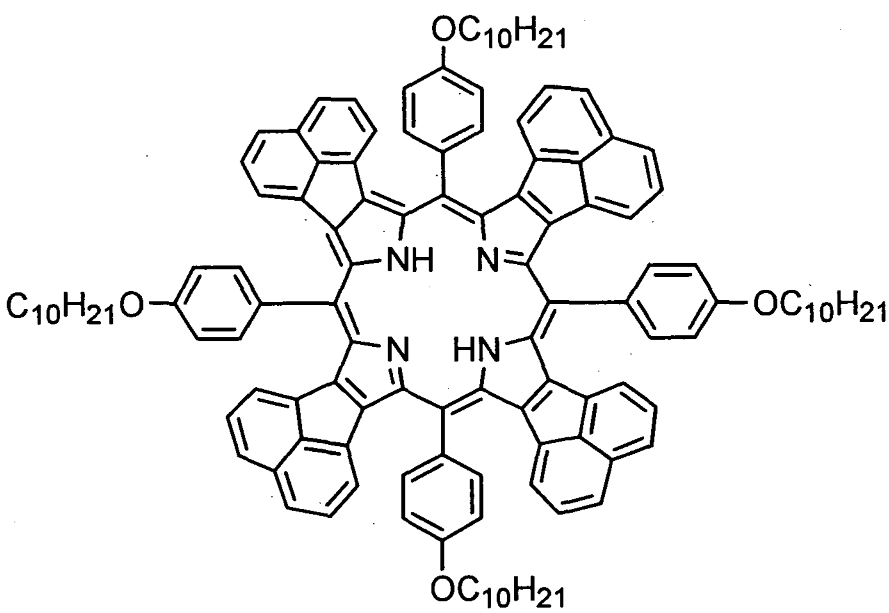

(1 − A)

The maximum absorption wavelength in chloroform of the compound was 567 nm, while the gram absorption coefficient was 113,000.

Analysis data: $^1$H-NMR

δ(ppm) : 8.65(d, 8H), 7.62(d, 8H), 7.42(d, 8H), 7.20(t, 8H), 5.88(d, 8H), 4.33(m, 8H), 2.03(m, 8H), 1.63(m, 8H), 1.5-1.2 (m, 48H), 0.93(t, 12H).

Example 1-2

In a nitrogen atmosphere, into a flask were introduced 1.5 g of the obtained compound represented by the above formula (1-A), 0.32 g of nickel (II) acetate and 300 ml of DMF. The resulting mixture was stirred at 120 °C for 2 hours, cooled down, and then extracted with chloroform and washed with water. The solvent was distilled off and then the residue was purified with a silica gel column. In the purification, a mixed solvent of toluene and hexane (mixing ratio: 7:3, volume) was used as a developing solution, whereby 0.4 g of a compound represented by the compound No. 1-6 (Table 1-1) was obtained.

The maximum absorption wavelength in chloroform of the compound was 536 nm, while the gram absorption

coefficient was 135,000.
Analysis data: $^1$H-NMR
δ(ppm) : 8. 36 (d, 8H), 7.61(d, 8H), 7.33 (d, 8H), 7.18(t, 8H), 5.80(d, 8H), 4.29(m, 8H), 2.01(m, 8H), 1.64(m, 8H), 1.5-1.2 (m, 48H), 0.92(t, 12H).

Synthesis Example 1-1

A metal-free tetra-acenaphthoporphyrin compound was synthesized in the same manner as in Example 1-1, except that p-propyloxybenzaldehyde was used instead of p-decyloxybenzaldehyde. Then, a compound represented by the following formula (1-B) was synthesized in the same manner as in Example 1-2, except that the metal-free tetra-acenaphthoporphyrin compound obtained as above was used instead of the compound represented by the above formula (1-A) and copper (II) acetate was used instead of nickel (II) acetate.
[Chemical Formula 11]

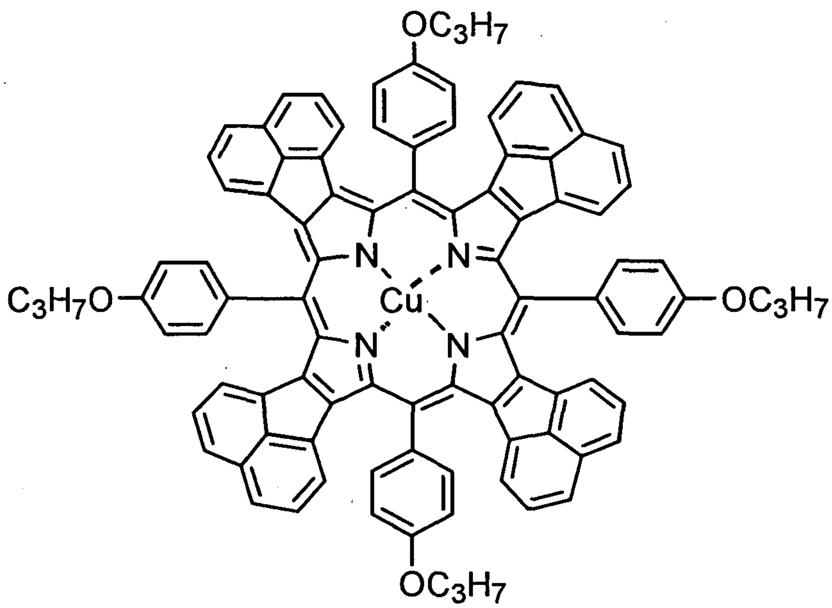

(1−B)

Example 1-3

Compounds represented by the compound Nos. 1-1, 1-2, 1-3, 1-4 and 1-7 (Table 1-1) were synthesized in the same manner as in Examples 1-1 and 1-2. With respect to compounds synthesized in Example 1-2 and Synthesis Example 1-1 along with these compounds, the maximum absorption wavelength (λmax), the gram absorption coefficient (eg) and solubility were measured. The results thereof are shown in Table 1-4.
λmax and eg are values in the chloroform solution, while the solubility is weight % based on acetone.

Note:

Solubility ○: completely dissolved
×: not completely dissolved

[Table 7]

| Table 1-4 | | | | | |
|---|---|---|---|---|---|
| | | | Solubility | | |
| Compound No. | λmax (nm) | εg | 0.1% | 0.2% | 1.0% |
| 1-1 | 553 | 162,000 | ○ | ○ | × |
| 1-2 | 553 | 154,000 | ○ | ○ | ○ |
| 1-3 | 553 | 122,000 | ○ | ○ | ○ |
| 1-4 | 552 | 115,000 | ○ | ○ | ○ |
| 1-6 | 536 | 135,000 | ○ | ○ | ○ |
| 1-7 | 552 | 122,000 | ○ | ○ | ○ |
| Synthesis Example 1-1 | 553 | 161,000 | × | × | × |

Example 1-4

A compound represented by the compound No. 1-4 (Table 1-1) was dissolved in a mixed solvent of acetone and toluene (weight ratio 50:50) such that the concentration was 0.1 weight % to give a dye-containing diluting agent. An acrylic adhesive and the dye-containing diluting agent (weight ratio 50:70) were mixed and applied by spin coating at 800 rpm for 30 seconds onto a glass plate, and the resulting material was dried to prepare an optical filter. The dry film thickness of the coating layer was 5 μm. In the above filter, light transmittance was measured by using a spectrophotometer (UV-3100, manufactured by Shimadzu Corporation). Light transmittance in a wavelength of 550 nm was 47.2%.

Example 2

Compound represented by the above formula (2-1) and optical filter using the compound

Example 2-1

In a nitrogen atmosphere, into a flask were introduced 2.2 g of 2-furaldehyde and 1L of chloroform. Then, 4.0 g of acenaphthopyrrole was added thereto and the resulting mixture was stirred. 0.55 g of $BF_3 \cdot Et_2O$ was added thereto and the mixture was stirred for 3 hours, and then 3.3 g of 2,3-dichloro-5,6-dicyano-1,4-benzoquinone was added and the mixture was stirred for 1 hour. The solvent was distilled off and then the residue was purified with a silica gel column. In the purification, chloroform (1% triethylamine contained) was used as a developing solution, whereby 4.8 g of a compound represented by the following formula (2-A) was obtained.

[Chemical Formula 12]

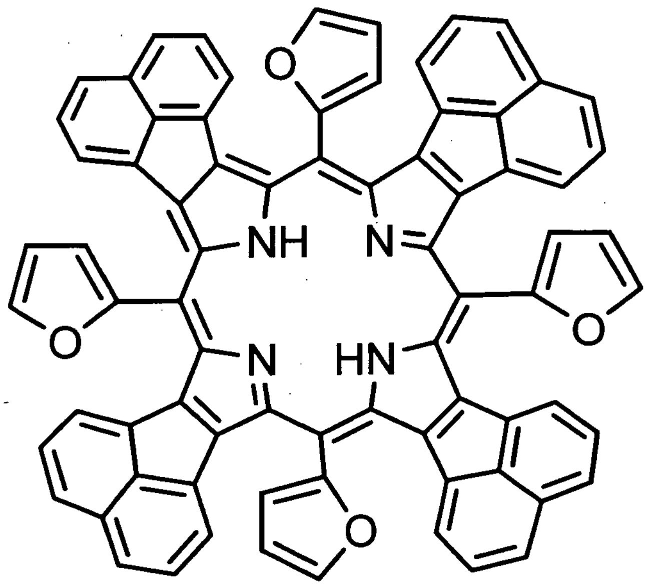

(2 − A)

The maximum absorption wavelength in chloroform of the compound was 563 nm, while the gram absorption coefficient was 110,000.
Analysis data

FD-MS:

calculation value: m/z=1071,
actual measurement value: 1071

Elemental analysis: C76H38N4O4·H2O

Calculation value (%) : C: 85.22, H: 3.58, N: 5.23
Actual measurement value (%): 84.98 3.39 5.20

Example 2-2

In a nitrogen atmosphere, into a flask were introduced 1.2 g of the obtained compound represented by the formula (2-A), 0.28 g of nickel (II) acetate and 300 ml of DMF. The resulting mixture was stirred at 120 ˚C for 2. 5 hours, cooled down, and then extracted with chloroform and washed with water. The solvent was distilled off and then the residue was purified with a silica gel column. In the purification, a mixed solvent of toluene and hexane (mixing ratio: 7: 3, volume) was used as a developing solution, whereby 0.3 g of a compound represented by the compound No. 2-13 (Table 2-2) was obtained.
The maximum absorption wavelength in chloroform of the compound was 534 nm, while the gram absorption coefficient was 121,000.
Analysis data

FD-MS:

calculation value: m/z=1127,
actual measurement value: 1127

Elemental analysis:

C76H36N4O4· Ni
C, 80.94; H, 3.22; N, 4.97; Ni, 5.20; O, 5.67

Calculation value (%): C: 80.94, H: 3.22, N: 4.97
Actual measurement value (%): 80.86 3.31 4.88

(Example 2-3)

A compound represented by the compound No. 2-12 (Table 2-2) synthesized in the same manner as in Examples 2-1 and 2-2 was dissolved in a mixed solvent of acetone and toluene (weight ratio 50:50) such that the concentration was 0.1 weight % to give a dye-containing diluting agent. An acrylic adhesive and the dye-containing diluting agent (weight ratio 50:70) were mixed and applied by spin coating at 800 rpm for 30 seconds onto a glass plate and the resulting material was dried to prepare an optical filter. The dry film thickness of the coating layer was 5 μm. In the above filter, light transmittance was measured by using a spectrophotometer (UV-3100, manufactured by Shimadzu Corporation). Light transmittance in a wavelength of 550 nm was 42.9%.

Example 3

Tetra-acenaphthoporphyrin compound, optical filter using the compound and plasma display using the optical filter

(Example 3-1)

Optical properties of tetra-acenaphthoporphyrin compound

A compound represented by the following formula (3-A) was synthesized in the same manner as in Examples 1-1 and 1-2.

With respect to the compounds represented by the compound Nos. 1-2, 1-3, 1-6 and 1-7 synthesized in Examples 1-2 and 1-3 (corresponding to compounds (3-B), (3-C), (3-E) and (3-F) respectively) along with the obtained compound (3-A), the maximum absorption wavelength and the ratio of the absorption coefficient at a wavelength of 550 nm to the absorption coefficient at a wavelength of 525 nm, which were measured in a chloroform solvent, are shown in Table 3-1.

[Chemical Formula 13]

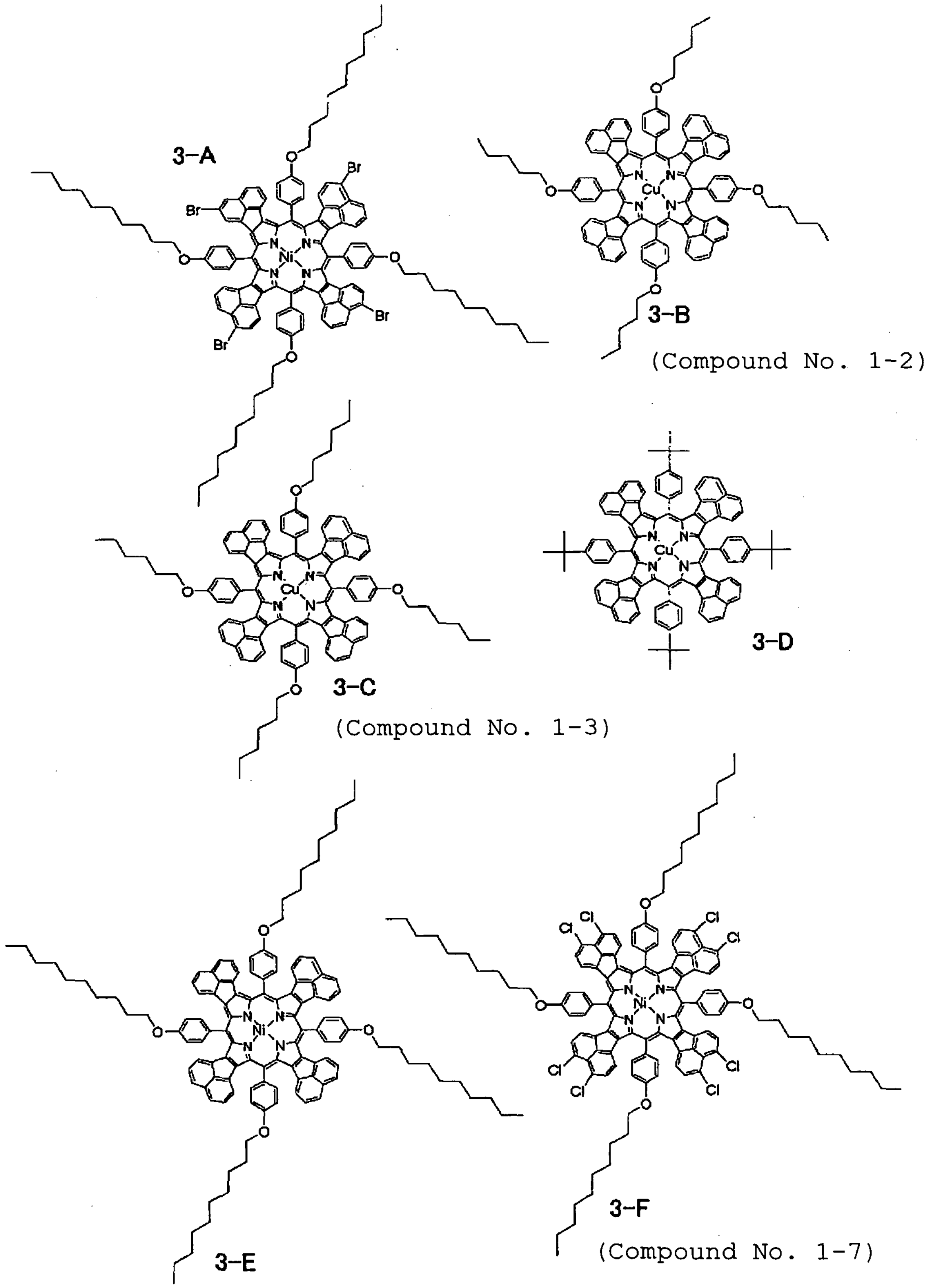

3-A

3-B

(Compound No. 1-2)

3-C

(Compound No. 1-3)

3-D

3-E

3-F

(Compound No. 1-7)

(Compound No. 1-6)

(Synthesis Example 3-1)

A compound represented by the above formula (3-D) was synthesized in the same manner as in Examples 1-1 and 1-2. With respect to the compound (3-D), the maximum absorption wavelength and the ratio of the absorption coefficient at a wavelength of 550 nm to the absorption coefficient at a wavelength of 525 nm, which were measured in the same manner as in Example 3-1, are shown in Table 3-1.

(Comparative Example 3-1)

With respect to a squarylium type compound (3-G), a cyanine type compound (3-H) and a pyrromethene type compound (3-I), the maximum absorption wavelength and the ratio of the absorption coefficient at a wavelength of 550 nm to the absorption coefficient at a wavelength of 525 nm, which were measured in the same manner as in Example 3-1, are shown in Table 3-1.

[Table 8]

Table 3-1

| Compound No. | Maximum Absorption Wavelength [nm] | Ratio of Absorption Coefficient at 525nm and 550nm [-] | Light Transmittance at 525nm [%] | Light Transmittance at 550nm [%] | T@550/T@525 Light Transmittance Ratio * [%] | Absorption Retention after Light resistance test for 250 hours [%] |
|---|---|---|---|---|---|---|
| 3-A | 546 | 1.7 | 62.4 | 48.6 | 78 | 95 |
| 3-B | 553 | 2.5 | 62.6 | 37.2 | 59 | 95 |
| 3-C | 553 | 2.3 | 69.1 | 49.8 | 72 | 80 |
| 3-D | 546 | 2.2 | 59.7 | 38.1 | 64 | 92 |
| 3-E | 536 | 1.2 | 61.7 | 57.5 | 93 | 97 |
| 3-F | 552 | 1.4 | 72.0 | 66.6 | 93 | 94 |
| | | | | | | |
| 3-G | 555 | 5.0 | 67.9 | 15.4 | 23 | 1 (Absorption retention rate after 24 hours) |
| 3-H | 553 | 2.0 | 52.5 | 31.7 | 60 | 10 |
| 3-I | 555 | 1.2 | 62.7 | 58.9 | 94 | 5 (Absorption retention rate after 24 hours) |
| *T@525 represents light transmittance at a wavelength of 525 nm, while T@550 represents light transmittance at a wavelength of 550 nm. | | | | | | |

(Example 3-2)

Optical properties and durability evaluation of optical filter

(Example 3-2-1)

Optical properties of optical filter

When the compound (3-A) was dispersed and dissolved in a solvent of ethyl acetate and toluene (weight ratio

50:50) and dried, a dye-containing diluting agent for an acrylic adhesive was prepared such that the dye concentration in the resin was 1000 (wt)ppm. Subsequently, the acrylic adhesive (a copolymer of butyl acrylate and acrylic acid (Product name: DBBOND, manufactured by Diabond Industry Co., Ltd.)) and a dye-containing diluting agent were mixed (weight ratio 80:20), and continuously applied on a mold releasing layer of a mold releasing film comprising a polyethylene terephthalate film and a silicone mold releasing layer according to a bar coating method, and the resulting material was dried. Then, the other mold releasing film was attached to the coating surface to prepare a dye-containing adhesive sheet having a thickness of 25 μm with mold releasing films having different mold releasing forces at both sides.

The mold releasing film on one side of the prepared adhesive sheet was peeled away and the resulting sheet was attached to a transparent acrylic resin (manufactured by Mitsubishi Rayon Co., Ltd., Acrylite, thickness: 2 mm), while the mold releasing film on the other side thereof was further peeled away and an ultraviolet blocking film (manufactured by Teijin Co., Ltd., HB Film, thickness: 75 μm) was attached to prepare an optical filter.

With respect to the obtained optical filter, the light transmittance in a wavelength of from 300 to 800 nm was measured by using a spectrophotometer (Product name: U-3400, manufactured by Hitachi, Ltd.) in accordance with JIS R3106. A percentage of light transmittance at a wavelength of 550 nm to light transmittance at a wavelength of 525 nm was obtained. The results thereof are shown in Table 3-1.

With respect to the compounds (3-B) to (3-F), optical filters were prepared in the same manner as in the compound (3-A). The measurement results of these optical filters are shown in Table 3-1.

(Comparative Example 3-2-1)

With respect to the compounds (3-G) to (3-I), optical filters were prepared in the same manner as in Example 3-2-1 and evaluated. The results thereof are shown in Table 3-1.

(Example 3-2-2)

Evaluation of light resistance of optical filter

With respect to the optical filters obtained from the compounds (3-A) to (3-F), environmental accelerated tests were carried out by artificial sunlight irradiation using a solar simulator (manufactured by Ushio Inc.). -A visible light ray in a wavelength of from 380 to 780 nm was irradiated on the ultraviolet blocking film of the prepared optical filter at 50 $mW/cm^2$ for 250 hours in a room at a temperature of 30 ± 10 ˚C and a humidity of 50 ± 20%. At this time, an absorption retention rate represented by the above equation (1) was obtained. The results thereof are shown in Table 3-1.

(Comparative Example 3-2-2)

With respect to optical filters obtained from the compounds (3-G) to (3-I), absorption retention rates were obtained in the same manner as in Example 3-2-2. The results thereof are shown in Table 3-1.

(Example 3-2-3)

Evaluation of durability of optical filter

With respect to the optical filters obtained from the compounds (3-A) and (3-B), environmental accelerated tests of high temperature, high-temperature/high-humidity and artificial sunlight irradiation were carried out. In the test, a constant temperature chamber at 80 ˚C (DN-61, manufactured by Yamato Scientific Co., Ltd.), a constant temperature and humidity chamber at 60 ˚C and 90 %RH (Product name, FX2200, manufactured by Kusumoto Chemicals, Ltd.) and a solar simulator (manufactured by Ushio Inc.) were respectively used. A visible light ray in a wavelength of from 380 to 780 nm was irradiated on a surface of the ultraviolet blocking film of the above optical filter at 50 $mW/cm^2$ for a predetermined time in a room at a temperature of 30 ± 10 ˚C and a humidity of 50 ± 20%.

The above optical filters were kept under each environmental condition for 500 hours and 1000 hours, and light transmittance of the optical filter was measured by using the spectrophotometer after 500 hours and 1000 hours. An absorption retention rate in a maximum absorption wavelength after each environmental accelerated test was obtained from the above equation (2). The results thereof are shown in Table 3-2.

[Table 9]

Table 3-2

| Compound No. | | Absorption retention rate (%) | |
|---|---|---|---|
| | | After 500 hours | After 1000 hours |
| 3-A | High temperature test | 88 | 89 |
| | High temperature/ High humidity test | 81 | 80 |
| | Artificial sunlight irradiation test | 94 | 92 |
| 3-B | High temperature test | 100 | 100 |
| | High temperature/ High humidity test | 91 | 90 |
| | Artificial sunlight irradiation test | 95 | 93 |

As shown from Table 3-2, in all environmental accelerated tests, the optical filters obtained from the compounds (3-A) and (3-B) had an absorption retention rate of not less than 80%, had a very excellent environmental resistance, and did not deteriorate performance over a long period of time even after 1000 hours had passed.

(Example 3-3)

Optical filter and plasma display equipped with the optical filter

(Example 3-3-1)

As a transparent substrate, a polyethylene terephthalate (PET) film (Product name: Tetoron Film, manufactured by Teijin Dupont Films Ltd., thickness: 75 μm) was used. On one surface of the transparent substrate were laminated a high refractive-index layer comprising oxide of indium and tin, and a metal thin film layer comprising silver using a DC magnetron sputtering method in the order of PET / a high refractive-index layer [film thickness: 40 nm] / a metal thin film layer [film thickness: 11 nm] / a high refractive-index layer [film thickness: 40 nm] / a metal thin film layer [film thickness: 14 nm] / a high refractive-index layer [film thickness: 95 nm] / a metal thin film layer [film thickness: 12 nm] / a high refractive-index layer [film thickness: 90 nm] to form a transparent electrically conductive thin film having 7 layers to give an electrically conductive layer. To form a high refractive-index layer comprising oxide of indium and tin, a sintered body ($In_2O_3$:$SnO_2$ = 90:10 (weight ratio)) of indium oxide and tin oxide was used as a target, while a mixed gas of argon and oxygen (total pressure: 266 mPa, oxygen partial pressure: 5 mPa) was used as a sputtering gas. Further, to form a metal thin film layer comprising silver, silver was used as a target, while an argon gas (total pressure: 266 mPa) was used as a sputtering gas.

A protective film having an adhesive layer (thickness: 3 μm) on a polymeric support of a polyethylene film (manufactured by Mitsui Chemicals, Inc., thickness: 50 μm) was attached to the electrically conductive layer formed as described above. The pressure at the time of attachment was 0.3 MPa.

Then, the compound (3-A) and a copper tetraazaporphyrin complex (maximum absorption wavelength: 595 nm) were dispersed and dissolved in a solvent of ethyl acetate and toluene (weight ratio 50:50) and dried such that the dye concentrations in the resin were 1000 (wt)ppm and 2500 (wt)ppm respectively, and MS-Yellow (manufactured by Mitsui Chemicals, Inc.) and MS-Blue (manufactured by Mitsui Chemicals, Inc.) having an absorption band in a visible light region as dyes for toning were added to prepare a dye-containing diluting agent for an acrylic adhesive. An acrylic adhesive (a copolymer of butyl acrylate and acrylic acid) and the dye-containing diluting agent were mixed (weight ratio 80:20), and continuously applied on a mold releasing layer of a mold releasing film comprising a polyethylene terephthalate film and a silicone mold releasing layer according to the bar coating method, and the resulting material was dried. Then, the other mold releasing film was attached to the coating surface to prepare a dye-containing adhesive sheet having a thickness of 25 μm with mold releasing films having different mold releasing forces at both sides.

The mold releasing film on one side of the above adhesive sheet was peeled away and the resulting sheet was attached to the transparent substrate where the above electrically conductive layer was not formed. Thus, a laminate comprising the mold releasing film / the dye-containing adhesive sheet / the transparent substrate / the electrically conductive layer / the protective film was prepared.

Then, the other mold releasing film was peeled away, and a film comprising the dye-containing adhesive sheet, the transparent substrate with the protective film attached thereto and the electrically conductive layer was attached to one surface of a thermally treated glass having a thickness of 3 mm. The pressure at the time of attachment was 0.3 MPa.

Furthermore, the protective film attached to the electrically conductive layer was peeled away, and an anti-reflection film (manufactured by Nippon Oils and Fats Co., Ltd., thickness: 105 μm) was attached. The pressure at the

time of attachment was 0.3 MPa.
In the above optical filter, light transmittance in a wavelength of from 300 to 800 nm was measured by using a spectrophotometer (Product name: U-3400, manufactured by Hitachi, Ltd.). As a result, a percentage of light transmittance at a wavelength of 550 nm to light transmittance at a wavelength of 525 nm of the obtained optical filter was 69%.

(Example 3-3-2)

An optical filter was prepared in the same manner as in Example 3-3-1, except that the compound (3-B) was used instead of the compound (3-A), and 550 (wt)ppm of the compound (3-B) and 1100 (wt)ppm of a copper tetraazaporphyrin complex (maximum absorption wavelength: 595 nm) were used respectively, and MS-Yellow (manufactured by Mitsui Chemicals, Inc.) and MS-Blue (manufactured by Mitsui Chemicals, Inc.) having an absorption band in a visible light range as dyes for toning were added. Its evaluation was carried out. A percentage of light transmittance at a wavelength of 550 nm to light transmittance at a wavelength of 525 nm of the obtained optical filter was 66%.

(Example 3-3-3)

The optical filter prepared in Example 3-3-1 was placed on a front surface of a plasma display panel such that the anti-reflection film was arranged in a human side.
The spectral radiance was measured in the middle of the screen of the plasma display equipped with the above optical filter by using a spectral radiance meter (MINOLTA CS-1000, manufactured by Konica Minolta Holdings, Incorporated). Using a three-band fluorescent tube (Product name: Highlumic N, HITACHI, manufactured by Hitachi Home & Life Solutions, Inc.), emission spectra of white color (W) and black color (BL) were measured in a room under 200 lux. From the spectra, the contrast in a bright place was calculated according to the following equation (3). In the formula, luminance of black image represents the luminance when the display screen displays black, while luminance of white image represents the luminance when the screen displays white. The results thereof are shown in Table 3-3.
$$\text{Contrast} = \text{Luminance of white image} / \text{Luminance of black image} \quad (3)$$

Furthermore, luminance spectra of white (W), red (R), green (G) and blue (B) of from 380 to 780 nm in a dark room were measured, while the color reproduction gamut was calculated from an area of a triangle obtained from the chromaticity coordinates of red, green and blue colors. The results thereof are shown in Table 3-3.
[Table 10]

Table 3-3

| | Compound No. | Contrast * | Color Reproduction Gamut * |
|---|---|---|---|
| Example 3-3-3 | 3-A | 143 | 102 |
| Example 3-3-4 | 3-B | 143 | 102 |
| Example 3-4-1 | 3-A | 148 | 104 |
| Example 3-4-2 | 3-B | 148 | 104 |
| Comparative Example 3-4-1 | - | 100 | 100 |
| * Contrast value is a relative value when the contrast of Comparative Example 3-4-1 was 100. The same is applied to the color reproduction gamut. | | | |

(Example 3-3-4)

The contrast and color reproduction gamut were calculated in the same manner as in Example 3-3-3 by placing the optical filter prepared in Example 3-3-2, instead of the optical filter prepared in Example 3-3-1, on a front surface of the plasma display panel.

(Example 3-4)

Optical filter and plasma display equipped with the optical filter

(Example 3-4-1)

An optical filter was prepared in the same manner as in Examples 3-3-1 and 3-3-2, except that the compound (3-A), a vanadyl porphyrin complex (maximum absorption wavelength: 500 nm) and a copper tetraazaporphyrin complex (maximum absorption wavelength: 595 nm) as dyes were used in amounts of 1000 (wt)ppm, 250 (wt)ppm and 2400 (wt)ppm respectively, and MS-Yellow (manufactured by Mitsui Chemicals, Inc.) and MS-Blue (manufactured by Mitsui Chemicals, Inc.) having an absorption band in a visible light region were added as dyes for toning. The optical filter was placed on the plasma display for evaluation. The results thereof are shown in Table 3-3.
Furthermore, a percentage of light transmittance at a wavelength of 550 nm to light transmittance at a wavelength of 525 nm of the obtained optical filter was 66%.

(Example 3-4-2)

An optical filter was prepared in the same manner as in Example 3-4-1, except that the compound (3-B), a vanadyl porphyrin complex (maximum absorption wavelength: 500 nm) and a copper tetraazaporphyrin complex (maximum absorption wavelength: 595 nm) as dyes were used in amounts of 550 (wt)ppm, 300 (wt)ppm and 1200 (wt)ppm respectively, and MS-Yellow (manufactured by Mitsui Chemicals, Inc.) and MS-Blue (manufactured by Mitsui Chemicals, Inc.) having an absorption band in a visible light region were added as dyes for toning. The optical filter was placed on the plasma display for evaluation. The results thereof are shown in Table 3-3.
Furthermore, a percentage of light transmittance at a wavelength of 550 nm to light transmittance at a wavelength of 525 nm of the obtained optical filter was 67%.

(Comparative Example 3-4-1)

The existing optical filter was used and evaluated in the same manner as in Examples 3-3-1 and 3-3-3. When the contrast and the color reproduction gamut under a three-band fluorescent tube of the plasma display equipped with the existing optical filter (Comparative Example 3-4-1) were both set as 100, the contrast and the color reproduction gamut under a three-band fluorescent tube of the plasma display equipped with the optical filter containing the tetraacenaphthoporphyrin compound of the present invention (Examples 3-3-3, 3-3-4, 3-4-1, 3-4-2) were shown in Table 3-3.
As a result, the contrast in a bright place was enhanced to 40% or more by placing the optical filter using the compound of the present invention, as compared to the existing optical filter. Further, it was confirmed that the color reproduction gamut was also improved. The above results are considered to be attributable to the fact that an unnecessary emission in the vicinity of 550 nm in the display unit was absorbed due to the optical filter of the present invention.

(Example 3-5)

Optical filter and plasma display equipped with the optical filter

(Example 3-5-1)

An adhesive sheet was prepared in the same manner as in Example 3-3-1, except that the compound (3-A), a vanadyl porphyrin complex (maximum absorption wavelength: 500 nm) and a copper tetraazaporphyrin complex (maximum absorption wavelength: 595 nm) as dyes were used in amounts of 1000 (wt)ppm, 250 (wt)ppm and 2400(wt) ppm respectively, and MS-Yellow (Product name, manufactured by Mitsui Chemicals, Inc.) and MS-Blue (Product name, manufactured by Mitsui Chemicals, Inc.) having an absorption band in a visible light region were used in amounts of 600 (wt)ppm and 1100 (wt)ppm respectively as dyes for toning.
A mold releasing film on one surface of the above adhesive sheet was peeled away, and the resulting sheet was attached to a PET film side of an electrically conductive copper mesh film (line width: 10 $\mu$m, pitch: 300 $\mu$m, bias angle: 45 degrees, film thickness: 150 $\mu$m) comprising an electrically conductive mesh layer and a PET film to prepare a laminate comprising the mold releasing film / the dye-containing adhesive sheet / the electrically conductive copper mesh film. Further, a near-infrared ray absorption film (Product name: CLEARAS NIR Film, manufactured by Sumitomo Osaka Cement Co., Ltd.) was formed on the electrically conductive mesh layer to prepare a laminate comprising the mold releasing film / the dye-containing adhesive sheet / the electrically conductive copper mesh film / the near-infrared ray absorption film.

Then, the mold releasing film was peeled away, and the above film comprising the dye-containing adhesive sheet / the electrically conductive copper mesh film / the near-infrared ray absorption film was attached to one surface of a heat treated glass having a thickness of 3 mm. Further, an anti-reflection film (manufactured by Nippon Oils and Fats Co., Ltd., thickness: 105 μm) was formed on the other surface of the thermally treated glass to prepare an optical filter. A percentage of light transmittance at a wavelength of 550 nm to light transmittance at a wavelength of 525 nm of the prepared optical filter was 60%. Furthermore, when the contrast in Comparative Example 3-4-1 was 100, the contrast of the prepared optical filter was 148.
Further, it was visually confirmed that the color of the prepared optical filter was a neutral color. The visible light transmittance as a film (measurement method: JIS R3106) was 37%.

(Example 3-5-2)

An optical filter was prepared and evaluated in the same manner as in Example 3-5-1, except that the compound (3-B), a vanadyl porphyrin complex (maximum absorption wavelength: 500 nm) and a copper tetraazaporphyrin complex (maximum absorption wavelength: 595 nm) as dyes were used in amounts of 550 (wt)ppm, 300 (wt)ppm and 1200 (wt) ppm respectively, and MS-Yellow (Product name, manufactured by Mitsui Chemicals, Inc.) and MS-Blue (manufactured by Mitsui Chemicals, Inc.) were used in amounts of 400 (wt)ppm and 550 (wt)ppm respectively as dyes for toning. A percentage of light transmittance at a wavelength of 550 nm to light transmittance at a wavelength of 525 nm of the prepared optical filter was 60%. Further, when the contrast in Comparative Example 3-4-1 was 100, the contrast of the prepared optical filter was 148.
Further, it was visually confirmed that the color of the prepared optical filter was a neutral color. The visible light transmittance as a film (measurement method: JIS R3106) was 34%.
From the above, the contrast in a bright place and the color reproduction gamut of the display unit can be improved by the optical filter containing the tetra-acenaphthoporphyrin compound of the present invention. Further, by excellent durability of the tetra-acenaphthoporphyrin to be used in the present invention, it is possible to provide clear and beautiful images over a long period of time as a display unit.

INDUSTRIAL APPLICABILITY

The optical filter of the present invention is properly used for all known display units. Since the optical filter selectively absorbs an unnecessary light in a wavelength of from 530 to 570 nm contained in external light and contains tetra-acenaphthoporphyrin having excellent durability, it is possible to display clear images with improved color purity which has excellent contrast and particularly [contrast in a bright place] in all places without deteriorating performance over a long period of time. For this reason, the industrial applicable value is high.

**Claims**

1. An optical filter containing at least one tetra-acenaphthoporphyrin compound.

2. The optical filter as set forth in claim 1, wherein said tetra-acenaphthoporphyrin compound has an absorption maximum in a wavelength range of from 530 to 570 nm and a ratio of an absorption coefficient at a wavelength of 550 nm to an absorption coefficient at a wavelength of 525 nm of not less than 1.2.

3. The optical filter as set forth in claim 1, wherein a percentage of light transmittance at a wavelength of 550 nm to light transmittance at a wavelength of 525 nm is not more than 85%.

4. The optical filter as set forth in claim 1, further containing a dye (A) having an absorption maximum in a wavelength range of from 440 to 510 nm and/or a dye (B) having an absorption maximum in a wavelength range of from 575 to 620 nm.

5. The optical filter as set forth in claim 1, comprising an electrically conductive layer (D) and/or a functional transparent layer (E).

6. The optical filter as set forth in claim 1, comprising a toning layer (F).

7. The optical filter as set forth in claim 1, wherein said tetra-acenaphthoporphyrin compound is a compound represented by the following general formula (3-1),

[Chemical Formula 1]

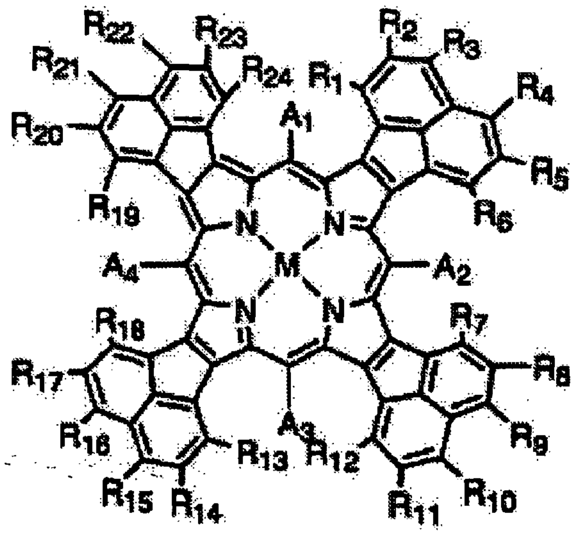

(3－1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $A_1$ to $A_4$ each independently represent a hydrogen atom, an alkyl group which may be substituted, an aryl group which may be substituted, a heteroaryl group which may be substituted, an alkenyl group which may be substituted or an alkynyl group which may be substituted; and M represents two hydrogen atoms, a divalent metal atom, or a trivalent or tetravalent metallic derivative.

**8.** A plasma display including the optical filter as set forth in claim 1.

**9.** A liquid crystal display including the optical filter as set forth in claim 1.

**10.** An organic electroluminescence display including the optical filter as set forth in claim 1.

**11.** An optical filter comprising a dye which has an absorption maximum in a wavelength range of from 530 to 570 nm and a ratio of an absorption coefficient at a wavelength of 550 nm to an absorption coefficient at a wavelength of 525 nm of not less than 1.2, and which has an absorption retention rate represented by the following equation (1) of not less than 80% when a visible light ray of from 380 to 780 nm is irradiated at 50 mW/cm$^2$ for 250 hours on an acrylic adhesive containing the dye,

$$\text{Absorption retention rate (\%)} = (T_0 - T_{max,1}) / (T_0 - T_{max,0}) \times 100 \qquad (1)$$

wherein To represents an average light transmittance (%) of light transmittances at from 380 to 780 nm that are constant in a wavelength range of 30 nm; $T_{max,0}$ represents the light transmittance (%) at a maximum absorption wavelength before a visible light ray is irradiated; and $T_{max,1}$ represents the light transmittance (%) at a maximum

absorption wavelength after a visible light ray is irradiated for 250 hours.

**12.** A tetra-acenaphthoporphyrin compound represented by the following general formula (1-1),

[Chemical Formula 2]

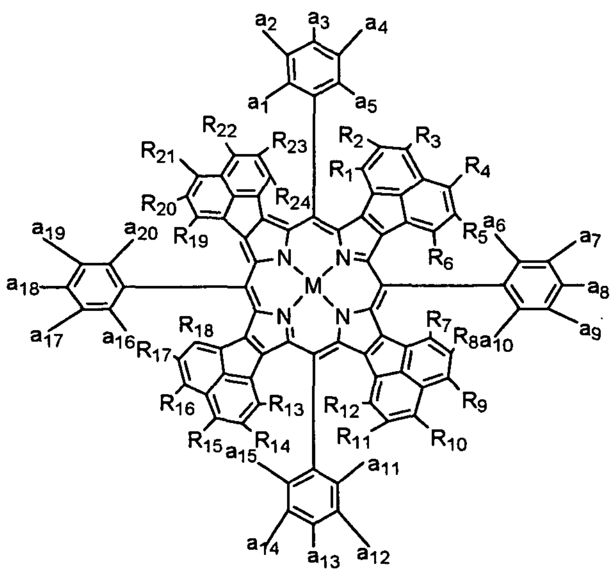

(1-1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $a_1$ to $a_{20}$ each independently represent a hydrogen atom, a halogen atom, an alkyl group which may be substituted, an aryl group which may be substituted or an alkoxy group which may be substituted, with the proviso that, in each of $a_1$ to $a_5$, $a_6$ to $a_{10}$, $a_{11}$ to $a_{15}$ and $a_{16}$ to $a_{20}$, one or more are an alkoxy group having 4 to 20 carbon atoms which may be substituted; and M represents two hydrogen atoms, a divalent metal atom, or a trivalent or tetravalent metallic derivative.

**13.** A tetra-acenaphthoporphyrin compound represented by the following general formula (2-1),

[Chemical Formula 3]

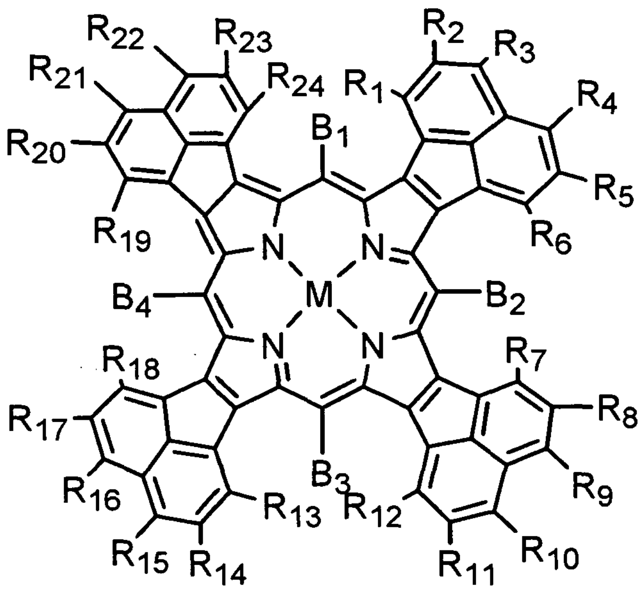

(2 − 1)

wherein $R_1$ to $R_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted; $B_1$ to $B_4$ each independently represent an alkenyl group which may be substituted, an alkynyl group which may be substituted or a heteroaryl group which may be substituted; and M represents two hydrogen atoms, a divalent metal atom, or a trivalent or tetravalent metallic derivative.

**14.** A tetra-acenaphthoporphyrin compound represented by the following general formula (2-2),

[Chemical Formula 4]

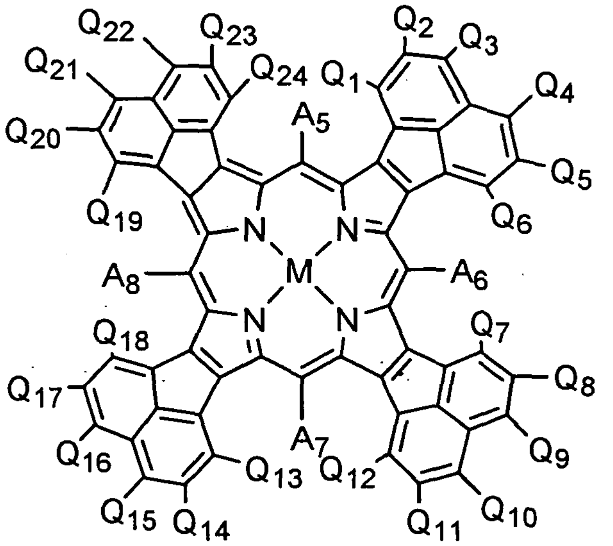

(2－2)

wherein $Q_1$ to $Q_{24}$ each independently represent a hydrogen atom, a halogen atom, a nitro group, a cyano group, a hydroxyl group, a formyl group, a carboxyl group, a sulfonic acid group, an alkyl group which may be substituted, an aryl group which may be substituted, a heteroaryl group which may be substituted, an alkoxy group which may be substituted, an aryloxy group which may be substituted, an alkylcarbonyl group which may be substituted, an arylcarbonyl group which may be substituted, an alkoxycarbonyl group which may be substituted, an aryloxycarbonyl group which may be substituted, an aminocarbonyl group which may be substituted, an amino group which may be substituted, an alkylthio group which may be substituted, an arylthio group which may be substituted, an aminosulfonyl group which may be substituted, an alkenyl group which may be substituted or an alkynyl group which may be substituted, with the proviso that one or more of $Q_1$ to $Q_{24}$ are a nitro group, a cyano group, a hydroxyl group, a carboxyl group, a sulfonic acid group, an alkoxycarbonyl group which may be substituted, an aryloxycarbonyl group which may be substituted, an aminocarbonyl group which may be substituted, an amino group which may be substituted, an aminosulfonyl group which may be substituted, an alkynyl group which may be substituted or a heteroaryl group which may be substituted; $A_5$ to $A_8$ each independently represent a hydrogen atom, an alkyl group which may be substituted or an aryl group which may be substituted; and M represents two hydrogen atoms, a divalent metal atom, or a trivalent or tetravalent metallic derivative.

**INTERNATIONAL SEARCH REPORT**

International application No.
PCT/JP2005/019475

A. CLASSIFICATION OF SUBJECT MATTER
*C07D487/22*(2006.01), *G02B5/00*(2006.01), *G02B5/22*(2006.01), *G02B5/30*(2006.01)

According to International Patent Classification (IPC) or to both national classification and IPC

B. FIELDS SEARCHED

Minimum documentation searched (classification system followed by classification symbols)
*C07D487/22*(2006.01), *G02B5/00*(2006.01), *G02B5/22*(2006.01), *G02B5/30*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2004-258514 A (Kasei Optonix, Ltd.), 16 September, 2004 (16.09.04), Full text; all drawings (Family: none) | 1-11 |
| A | JP 2002-138203 A (Sumitomo Osaka Cement Co., Ltd.), 14 May, 2002 (14.05.02), Full text; all drawings (Family: none) | 1-11 |
| P,A | JP 2005-120303 A (Mitsui Chemicals, Inc.), 12 May, 2005 (12.05.05), Full text; all drawings (Family: none) | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
26 January, 2006 (26.01.06)

Date of mailing of the international search report
07 February, 2006 (07.02.06)

Name and mailing address of the ISA/
Japanese Patent Office

Facsimile No.

Authorized officer

Telephone No.

Form PCT/ISA/210 (second sheet) (April 2005)

**INTERNATIONAL SEARCH REPORT**

International application No.
PCT/JP2005/019475

C (Continuation). DOCUMENTS CONSIDERED TO BE RELEVANT

| Category* | Citation of document, with indication, where appropriate, of the relevant passages | Relevant to claim No. |
|---|---|---|
| A | JP 9-197301 A (Denso Corp.), 31 July, 1997 (31.07.97), Full text; all drawings (Family: none) | 1-11 |
| A | JP 2-113202 A (Optical Coatings Japan), 25 April, 1990 (25.04.90), Full text; all drawings (Family: none) | 1-11 |
| A | JP 2003-207608 A (Sony Corp.), 25 July, 2003 (25.07.03), Full text; all drawings (Family: none) | 1-11 |
| A | JP 5-163408 A (Daicel Chemical Industries, Ltd.), 29 June, 1993 (29.06.93), Full text; all drawings (Family: none) | 11 |
| X<br>Y | JP 10-166732 A (Mitsui Chemicals, Inc.), 23 June, 1998 (23.06.98), Claims; Par. Nos. [0054] to [0063] (Family: none) | 13,14<br>12 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11259006 A
- JP 58153904 A
- JP 59221943 A
- JP 60022102 A
- JP 60065050 A
- JP 5316329 A
- JP 2002363434 A
- JP 2003167118 A
- JP 3004222 B
- JP 2002040233 A


### Non-patent literature cited in the description


- *J. Am. Chem. Soc.,* 1996, vol. 118, 8767-8768